# FASCICULE DE BREVET EUROPEEN

(11) **EP 0 596 037 B1**
(45) Date de publication et mention de la délivrance du brevet: **02.05.2001**
(21) Numéro de dépôt: 92917875.4
(22) Date de dépôt: 22.07.1992
(51) Int. Cl.: A61K 49/10, C07F 13/00

(54) **PRODUIT RADIOPHARMACEUTIQUE AYANT NOTAMMENT UN TROPISME CEREBRAL COMPORTANT UN COMPLEXE NITRIRO D'UN METAL DE TRANSITION, ET SON PROCEDE DE PREPARATION**
Uebergangsmetall-Nitridokomplex enthaltendes Radiopharmazeutisches Produkt mit einer Neigung zum Gehirn, Verfahren zu deren Herstellung
RADIOPHARMACEUTICAL PRODUCT HAVING IN PARTICULAR A CEREBRAL TROPISM COMPRISING A TRANSITION METAL NITRIDE COMPLEX, AND PREPARATION METHOD THEREFOR

(30) Priorité: 22.07.1991 FR 9109231
(43) Date de publication de la demande: 11.05.1994
(73) Titulaire: CIS BIO INTERNATIONAL, 91400 Saclay (FR)
(72) Inventeur: PASQUALINI, Roberto, F-92140 Clamart (FR); BELLANDE, Emmanuel, F-91160 Champlan (FR); COMAZZI, Véronique, F-92130 Issy-les-Moulineaux (FR); LAINE, Jacques, F-92160 Antony (FR)
(74) Mandataire: Des Termes, Monique
(86) Numéro de dépôt international: FR9200718
(87) Numéro de publication internationale: WO9301839

(56) Documents cités:
- WO-A-91/19516
- CHEMICAL ABSTRACTS, vol. 112, 1990, Columbus, Ohio, US; abstract no. 231908z, ABRAM, S. ET AL 'BLOOD CELL LABELLING EXPERIMENTS WITH LIPOPHILIC TECHNETIUM COMPLEXES' page 288 ;
- CHEMICAL ABSTRACTS, vol. 107, 1987, Columbus, Ohio, US; abstract no. 92985d, 'LIPOPHILIC TECHNETIUM COMPLEXES. IV. NEUTRAL, LIPID-SOLUBLE TECHNETIUM COMPLEXES WITH DITHIOLIGANDS CONTAINING Tc:O AND Tc:N CORES. AN IN VITRO STUDY' page 369 ;
- CHEMICAL ABSTRACTS, vol. 101, 1984, Columbus, Ohio, US; abstract no. 16223q, ABRAM, U. ET AL 'LIPOPHILIC TECHNETIUM COMPLEXES. PART I. DITHIOCARBAMATO COMPLEXES OF TECHNETIUM(III) AND -(V)' page 579 ;
- CHEMICAL ABSTRACTS, vol. 107, 1987, Columbus, Ohio, US; abstract no. 205147c, BALLINGER, J.R. ET AL 'TECHNETIUM-99M DIETHYL DITHIOCARBAMATE (DDC): COMPARISON WITH THALLIUM-201 DDC AS AN AGENT FOR BRAIN IMAGING' page 451 ;
- CHEMICAL ABSTRACTS, vol. 102, 1985, Columbus, Ohio, US; abstract no. 145416, BALDA, J. ET AL 'SUBSTITUTION REACTIONS OF 99mTc-NITRIDOTETRACHLOROTECHNATE(1-)ION A ROUTE TO A NEW CLASS OF 99mTc-RADIOPHARMACEUTICALS' page 271 ;
- J. CHEM. SOC., DALTON TRANS. no. 12, 1990, pages 3729 - 3733 DUATTI, A. ET AL
- JOURNAL OF THE CHEMICAL SOCIETY, DALTON TRANSACTIONS no. 10, 1972, pages 1079 - 1082 FLETCHER, S.R. ET AL 'CRYSTAL AND MOLECULAR STRUCTURE OF THE SQUARE-PYRAMIDAL COMPLEX NITRIDOBIS(NN-DIE THYLDITHIOCARBAMATO)RHENIUM(V)'
- BULLETIN OF THE CHEMICAL SOCIETY OF JAPAN vol. 61, no. 3, 1988, pages 693 - 698 SHIMURA, Y. 'A QUANTITATIVE SCALE OF THE SPECTROCHEMICAL SERIES FOR THE MIXED LIGAND COMPLEXES OF d6 METALS'

## Description

La présente invention a pour objet un produit radiopharmaceutique ayant notamment un tropisme cérébral, qui comprend un complexe nitruro d'un métal de transition comportant une partie M ≡ N, dans laquelle M représente le métal de transition.

On précise que l'on entend par métal de transition, un métal dont la couche d est partiellement remplie dans le degré d'oxydation usuel de ce métal. Il s'agit des éléments remplissant les périodes III à XII du tableau périodique des éléments à dix-huit colonnes.

A titre d'exemple de tels métaux, on peut citer Tc, Ru, Co, Pt, Fe, Os, Ir, W, Re, Cr, Mo, Mn, Ni, Rh, Pd, Nb et Ta.

Des complexes nitruro de technétium ont été décrits par J. Baldas et Col. dans les documents suivants : demande de brevet internationale WO-85/03 063, et le livre "Technetium in Chemistry and Nuclear Medicine", Ed. M. Nicolini, G. Bandoli, U. Mazzi, Cortine Int. Verone, 1986, pages 103 à 108.

Dans ces documents, on décrit la préparation de complexes nitruro de technétium par réaction de substitution sur [^{99m}TcNCl₄] et il est indiqué que ces complexes peuvent être utilisés comme produits radiopharmaceutiques, mais ces documents ne donnent aucun résultat probant sur la fixation de ces complexes dans le corps, et ne donnent donc aucune indication sur leurs tropismes, vis-à-vis de certains organes, notamment du cerveau. Par ailleurs, les ligands testés sont principalement le diéthylènetriaminepentacétate (DTPA), le méthylène disphosphonate (MDP), la cystéine (CYS), le gluconate (GLUC), l'éthane-1-hydroxy-1,1-diphosphonate (EHDP), le N-(2,6-diméthylphénylcarbamoylméthyl)iminodiacétate (HIDA), le N-(2,6-diisopropylphénylcarbamoylméthyl)iminodiacétate (PIPIDA) et le dimercaptosuccinate (DMSA).

Des produits radiopharmaceutiques, utilisables pour l'imagerie du cerveau doivent présenter certaines caractéristiques qui sont tout d'abord de pouvoir traverser la barrière hématoencéphalique, de se fixer et de s'accumuler dans le cerveau en un temps relativement court à une concentration élevée, et d'y rester pendant une durée suffisante pour que l'on puisse effectuer un examen.

Des complexes neutres de technétium présentant ces caractéristiques sont décrits dans les documents EP-A- 0 394 126, EP-A- 163 119, EP-A- 0 279 417, EP-A- 0 194 843 et FR-A- 2 651 232.

Dans les documents EP-A- 194 843, EP-A- 163 119, FR-A- 2 651 232 et EP-A- 0 394 126, les complexes utilisés comprennent des ligands à groupes amino, céto ou thiol qui peuvent être couplés à un ion métallique radioactif tel que Tc0. Il ne s'agit donc pas de complexes nitruro du technétium.

On connaît toutefois par le document EP-A- 0 279 417 des ligands diaminodithiols avec des substituants ester qui peuvent être couplés à un motif Tc≡N pour former des complexes nitruro utilisables pour l'imagerie du cerveau.

On connaît encore, par le document WO-90/06137 des complexes nitruro de métal de transition, utilisables comme produit radiopharmaceutique à tropisme cardiaque, mais les résultats de biodistribution obtenus- avec les complexes des exemples de ce document montrent qu'ils sont peu retenus par le cerveau.

Cependant, à la suite de recherches effectuées sur d'autres produits radiopharmaceutiques du même type, on a trouvé que certains d'entre eux présentaient des propriétés satisfaisantes pour une utilisation comme produits de diagnostic ou de thérapie ayant un tropisme cérébral, par exemple pour la scintigraphie du cerveau.

Aussi, la présente invention a pour objet ces nouveaux produits radio pharmaceutiques qui comprennent un complexe d'un métal de transition répondant à la formule :

(M≡N) L¹ L² (I)

dans laquelle L¹ et L² qui peuvent être identiques ou différents, répondent à la formule : 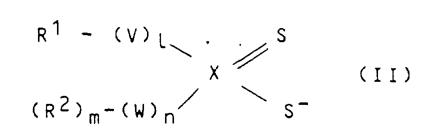 dans laquelle R¹ et R² qui peuvent être identiques ou différents, représentent un radical alkyle, linéaire ou ramifié de 1 à 10 atomes de carbone, non substitué ou substitué par au moins un groupement choisi parmi les groupements -O-R³, -OOCR³, -COOR³,-OCNR⁴R⁵ ou -NR⁴R⁵ dans lesquels R³ est un radical alkyle linéaire ou ramifié de 1 à 5 atomes de carbone, et R⁴ et R⁵ qui peuvent être identiques ou différents, sont des atomes d'hydrogène ou des radicaux alkyle, linéaires ou ramifiés, de 1 à 5 atomes de carbone, ou dans laquelle R¹ et R² forment ensemble un cycle hydrocarboné contenant éventuellement un ou plusieurs hétéroatomes, non substitué ou substitué par au moins un groupement choisi parmi -OR³, -COOR³, -OOCR³, -OCNR⁴R⁵ ou -NR⁴R⁵ dans lesquels R³, R⁴ et R⁵ sont tels que définis ci-dessus,
- V et W qui peuvent être identiques ou différents, représentent O, S ou Se,
- X représente C, N-C, P ou As, et
- L, m et n, qui peuvent être identiques ou différents, sont égaux à 0 ou à 1, m et n étant égaux à 0 lorsque X représente C, et m étant égal à 1 lorsque X représente N-C, P ou As,
à condition que
1°) lorsque X représente -N-C-, R¹ ou R² soit un radical alkyle substitué par au moins un groupement -COOR³, ou R¹ et R² forment ensemble un cycle hydrocarboné substitué, contenant éventuellement un ou plusieurs hétéroatomes, et
2°) Lorsque X représente P avec V et W représentant O, R¹ ou R² soit un radical alkyle substitué par au moins un groupement choisi parmi les groupements OR³, OOCR³, COOR³, OCNR⁴R⁵ ou NR⁴R⁵ avec R³, R⁴ et R⁵ tels que définis ci-dessus, ou R¹ et R² forment ensemble un cycle hydrocarboné substitué, contenant éventuellement un ou plusieurs hétéroatomes.

Lorsque R¹ et R² forment ensemble avec X un cycle hydrocarboné contenant éventuellement un ou plusieurs hétéroatomes, le nombre d'atomes de ce cycle, y compris l'atome de X, est généralement de 5 à 7.

Dans les produits radiopharmaceutiques de l'invention, le complexe nitruro de métal de transition peut être de différents types.

Ainsi, selon un premier mode de réalisation de l'invention, les ligands L¹ et L² sont du type dithiocarbamate et répondent à la formule : 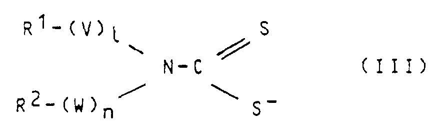 dans laquelle R¹ ou R² est un radical alkyle substitué par -COOR³.

De préférence, l et n sont égaux à 0. Généralement, R¹ est un radical alkyle substitué par -COOR³, et R² est un radical alkyle ou un radical alkyle substitué par -COOR³.

A titre d'exemple, L¹ et L² peuvent répondre aux formules suivantes : 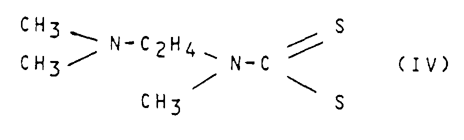 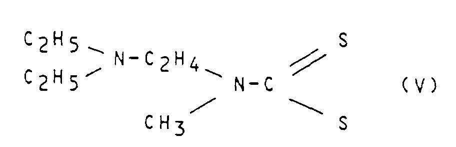 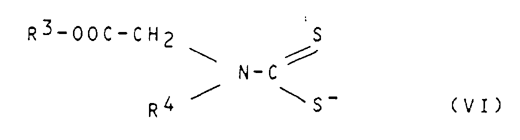 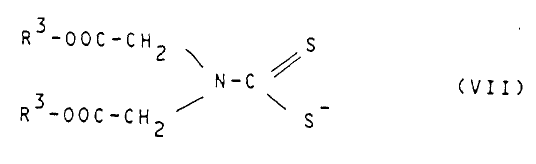 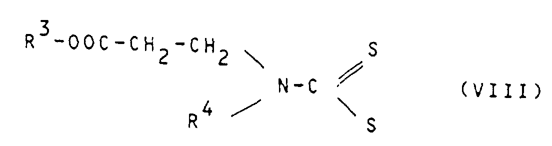 dans lesquelles R³ est le radical méthyle ou éthyle et R⁴ est le radical méthyle, éthyle ou propyle.

Selon un second mode de réalisation de l'invention, les ligands L¹ et L² répondent à la formule : 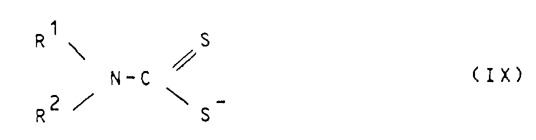 dans laquelle R¹ et R² forment ensemble un cycle hydrocarboné substitué par un groupement COOR³.

Le cycle hydrocarboné formé par 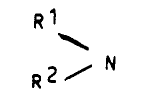 comprend par exemple 5 à 7 atomes y compris l'atome d'azote.

A titre d'exemple d'un tel ligand, on peut citer celui répondant à la formule :

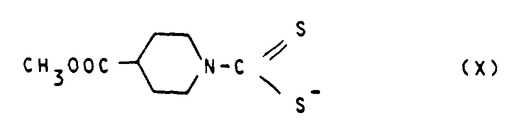

Selon un troisième mode de réalisation de l'invention, les ligands L¹ et L² répondent à la formule : 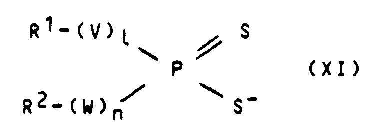 dans laquelle R¹ et R² sont des radicaux alkyle éventuellement substitués.

Lorsque l et n sont égaux à 0, les ligands L¹ et L² sont du type dithiophosphinates.

A titre d'exemple de tels ligands dithiophosphinates, on peut citer ceux répondant aux formules suivantes :

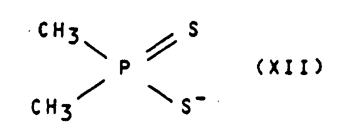

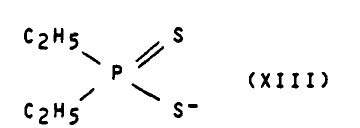

Dans ce troisième mode de réalisation, L¹ et L² peuvent être identiques ou différents.

Dans ce troisième mode de réalisation de l'invention, lorsque l=n=1, les ligands L¹ et L² sont du type dithiophosphates et répondent à la formule : 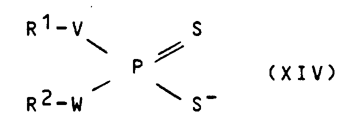 dans laquelle V et W sont O, S ou Se, par exemple O, et R¹ et R² ont la signification donnée ci-dessus.

Selon un quatrième mode de réalisation de l'invention, L¹ et L² sont du type dithiocarboxylate et ils répondent à la formule :

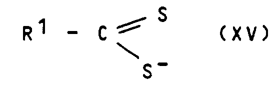

Dans les complexes de l'invention, le métal de transition utilisé dépend en particulier de l'application du produit radiopharmaceutique.

Ainsi, lorsqu'on veut utiliser le produit pour le diagnostic, on utilise un métal de transition radioactif, ayant une période relativement courte, par exemple le technétium ⁹⁹m.

Dans le cas où l'on veut utiliser le produit radiopharmaceutique pour la thérapie, on utilise un métal de transition émettant un rayonnement efficace pour la thérapie, et ayant une durée de vie plus longue tel que le rhénium, par exemple Re-186 ou Re-188.

Les complexes nitruro de technétium utilisés dans l'invention, peuvent être préparés par le procédé de Baldas. Toutefois, on préfère généralement les préparer par un procédé plus simple, facile à mettre en oeuvre dans un service hospitalier et conduisant à des rendements élevés.

Ce procédé comprend les étapes successives suivantes :
1°) faire réagir en solution un composé oxygéné d'un métal de transition M avec :
   a) un premier réactif choisi dans le groupe des phosphines et polyphosphines aliphatiques et aromatiques, substituées ou non substituées, et
   b) un second réactif choisi parmi les azotures d'ammonium ou de métaux pharmaceutiquement acceptables et Les ligands azotés comportant un motif 〉N-N〈 dans lesquels les N sont reliés à des atomes d'hydrogène et/ou à des groupements organiques monovalents,ou dans lequel l'un des N est relié à l'atome de carbone d'un groupe organique bivalent par l'intermédiaire d'une double liaison et l'autre N est relié à des atomes d'hydrogène et/ou à des groupements organiques monovalents, et
2°) faire réagir le produit intermédiaire obtenu dans la première étape avec un composé répondant à la formule : 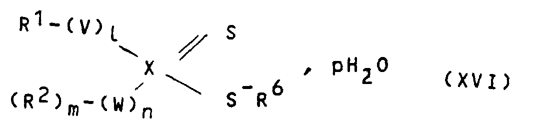 dans laquelle R¹, R², V, W, l, m, n et X ont la signification donnée ci-dessus,
   R⁶ est un ion de métal alcalin, H⁺ ou NH₄⁺, et p est égal à 0 ou est un nombre entier allant de 1 à 5.

Lorsque l'on met en oeuvre ce procédé en utilisant comme métal de transition du technétium, le composé oxygéné du métal de transition peut être du pertechnétate de métal alcalin ou d'ammonium. Dans le cas où le métal de transition est le rhénium, on peut utiliser un perrhénate de métal alcalin ou d'ammonium.

Dans la première étape du procédé, on prépare ainsi un premier complexe nitruro de technétium que l'on fait réagir ensuite avec le composé de formule (XVI) pour échanger le premier et le second réactifs par ce composé.

Pour réaliser la réaction, on peut introduire aseptiquement le premier et le second réactifs dans un récipient, puis ajouter la quantité requise de composé oxygéné de métal de transition, par exemple de pertechnétate de technétium ^{99m}, après avoir ajusté le pH à une valeur appropriée par addition d'acide ou de base. On peut ensuite effectuer la réaction à la température ambiante ou à une température supérieure allant de 50 à 100°C. La température et le pH utilisés dépendent en particulier du second réactif. Généralement on opère entre pH 2 et 7.

Dans la première étape, on peut utiliser le premier et le second réactifs sous la forme de solutions aqueuses, alcooliques ou hydroalcooliques et ajouter simplement ces solutions au composé oxygéné du métal de transition.

Dans la deuxième étape, on fait réagir le produit obtenu dans la première étape avec le composé de formule (XVI) en solution aqueuse, généralement à un pH supérieur à 7, par exemple dans un tampon carbonate-bicarbonate de sodium.

Dans cette deuxième étape, on peut aussi utiliser une solution alcoolique ou hydroalcoolique du composé (XVI).

Le premier réactif qui permet d'obtenir la formation d'un complexe nitruro est un ligand organique à atome de phosphore donneur d'électrons choisi parmi les phosphines et les polyphosphines aliphatiques et aromatiques, substituées ou non substituées.

Les phosphines utilisables peuvent répondre à la formule : 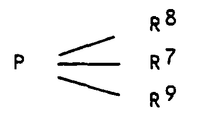 dans laquelle R⁷, R⁸ et R⁹ qui peuvent être identiques ou différents, représentent un atome d'hydrogène, un radical alkyle, un radical aryle, un radical alcoxy ou un radical alkyle ou aryle substitué par un groupement choisi parmi les radicaux amino, amido, cyano, sulfonate, chloro, fluoro ou carboxylate.

A titre d'exemple de phosphines de ce type, on peut citer la triphénylphosphine, la triphénylphosphine trisulfonée, la diéthylphénylphosphine, la triéthylphosphine, la triméthylphosphine et la tris(2-cyanoéthyl)phosphine P(CH₂-CH₂CN)₃.

Dans la première étape, on peut utiliser comme second réactif, soit un azoture de métal alcalin ou d'ammonium, par exemple de l'azoture de sodium, soit un ligand azoté comportant le motif N-N comme dans l'hydrazine et ses dérivés. On peut utiliser de nombreux ligands azotés de ce type. Généralement, on préfère utiliser comme ligand azoté, l'acide dithiocarbazique ou un dérivé de celui-ci, comme il est décrit dans WO-89/00608, ou des ligands azotés répondant à la formule : 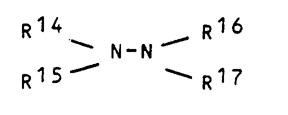
dans laquelle R¹⁴, R¹⁵ et R¹⁶ qui peuvent être identiques ou différents, représentent un atome d'hydrogène, un radical alkyle, un radical aryle, un radical alcoxy, un radical alkyle substitué par au moins un groupement choisi parmi les radicaux hydroxy, carboxy, amino, amido et mercapto ou un radical aryle substitué par au moins un groupement choisi parmi les atomes d'halogène et les radicaux alcoxy, hydroxy, amino, mercapto et amino substitué par au moins un radical alkyle,
   R¹⁷ représente un radical répondant aux formules

   -SO₂R¹⁸ ou -CO R¹⁹
dans lesquelles R¹⁸ est un groupe phényle non substitué ou substitué par au moins un substituant choisi parmi les atomes d'halogène et les radicaux alkyle, et
   R¹⁹ est un atome d'hydrogène, -NH₂ ou un radical choisi parmi les radicaux alkyle, les radicaux alkyle substitués par au moins un groupement choisi parmi les groupements cyano, pyridyle et -CO-NH-NH₂, le radical phényle, le radical phényle substitué par au moins un substituant choisi parmi -OH, NH₂ et les radicaux alkyle et alcoxy.

Dans cette formule, les radicaux alkyle et alcoxy peuvent être des radicaux linéaires ou ramifiés, de préférence de 1 à 4 atomes de carbone, par exemple le radical méthyle ou méthoxy.

Les radicaux aryle sont des radicaux dérivés d'un noyau par élimination d'un atome d'hydrogène, par exemple les radicaux phényle et naphtyle.

L'utilisation des ligands répondant à la formule précitée est intéressante car elle permet de réaliser la première étape à la température ambiante pour former le produit intermédiaire, et de préparer ensuite un produit radiopharmaceutique d'une pureté radiochimique d'au moins 95%, par réaction de ce produit intermédiaire avec un deuxième ligand.

A titre d'exemple de dérivés d'acide dithio carbazique utilisables comme ligand azoté, on peut citer le S-méthyldithiocarbazate, le S-méthyl-N-méthyldithiocarbazate, l'alpha-N-méthyl-S-méthyl bêta-N-pyridylméthylène dithiocarbazate, le S-méthyl-bêta-N-(2-hydroxyphényl)méthylène dithiocarbazate et le alpha-N-méthyl-S-méthyl-bêta-N-(2-hydroxyphényl)méthylène dithiocarbazate.

De préférence, lorsqu'on veut réaliser la réaction à la température ambiante, le premier ligand azoté répond à la formule précitée : 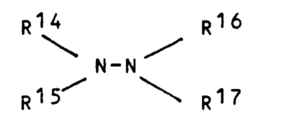 dans laquelle R¹⁴, R¹⁵ et R¹⁶ sont des atomes d'hydrogène, R¹⁷ représente le radical de formule SO₂R¹⁸ avec R¹⁸ ayant la signification donnée ci-dessus, ou le radical de formule

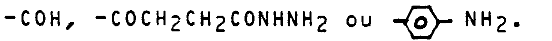

A titre d'exemples, lorsque R¹⁴, R¹⁵ et R¹⁶ sont des atomes d'hydrogène et R¹⁷ représente SO₂R¹⁸, R¹⁸ peut être le radical phényle, le radical p-méthylphényle, le radical trichloro-1,3,5 phényle ou le radical triméthyl-1,3,5 phényle.

On peut aussi préparer les produits radiopharmaceutiques de L'invention par un second procédé comprenant les étapes suivantes :
1°) faire réagir en solution un composé oxygéné du métal de transition M avec
   a) un ligand azoté constitué soit par un azoture d'ammonium ou de métal pharmaceutiquement acceptable, soit par un composé azoté comportant un motif 〉N-N〈 dans lequel les N sont reliés à des atomes d'hydrogène et/ou à des groupements organiques monovalents, ou dans lequel l'un des N est relié à l'atome de carbone d'un groupement organique bivalent par l'intermédiaire d'une double Liaison et l'autre N est relié à des atomes d'hydrogène et/ou à des groupements organiques monovalents, et
   b) un agent réducteur constitué, soit par du dithionite d'ammonium ou d'un métal pharmaceutiquement acceptable, soit par de l'étain (II) présent sous forme ionique dans la solution, et
2°) faire réagir le produit intermédiaire obtenu dans la première étape avec un composé répondant à la formule : 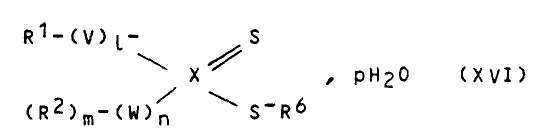 dans laquelle R¹, R², V, W, l, m, n et X ont la signification donnée ci-dessus,
   R⁶ est un ion de métal alcalin, H⁺ ou NH₄⁺, et p est égal à 0 ou est un nombre entier allant de 1 à 5.

Dans ce second procédé, on utilise donc à la place des phosphines ou polyphosphines du premier procédé, un agent réducteur constitué, soit par de l'étain (II), soit par un dithionite d'ammonium ou d'un métal pharmaceutiquement acceptable.

Ceci simplifie considérablement la mise au point de nouveaux produits radiopharmaceutiques tels que les produits à base de technétium, car la toxicité et les effets biologiques de l'étain comme le chlorure stanneux, sont bien connus dans ce domaine, ce qui n'est pas du tout le cas pour les phosphines et polyphosphines du premier procédé.

En effet, l'étain est utilisé depuis longtemps pour la préparation de complexes de technétium du type pyrophosphate, méthylènediphosphonate et hydroxymethylènediphosphonate.

De plus, les composés d'étain (II) et les dithionites sont généralement solubles dans l'eau, ce qui simplifie la préparation du complexe nitruro de métal de transition puisque l'on peut opérer en solution aqueuse, c'est-à-dire dans un milieu approprié pour l'administration à l'homme et aux êtres vivants.

Dans ce second procédé, on peut effectuer les deux réactions successivement ou simultanément.

Toutefois, on préfère généralement réaliser les deux réactions successivement.

Ce second procédé peut être mis en oeuvre de différentes façons qui dépendent en particulier de l'agent réducteur utilisé ainsi que du choix du ligand azoté et du composé (XVI) utilisé dans la deuxième étape.

Selon un premier mode de réalisation de ce second procédé, on utilise comme agent réducteur l'étain (II) et on l'introduit dans la solution à partir d'un ou plusieurs réactifs capables de le maintenir sous forme ionique en présence du ligand azoté et éventuellement du composé (XVI) pour éviter la précipitation des complexes d'étain susceptibles de se former avec le ligand azoté ou le composé (XVI).

Ainsi, on peut introduire l'étain sous la forme de sel d'étain (II) lorsque l'anion de ce sel d'étain a un meilleur pouvoir complexant sur l'étain que les autres réactifs présents dans la solution, c'est-à-dire le ligand azoté et éventuellement le composé (XVI).

A titre d'exemple, le sel d'étain peut être le tartrate ou l'oxalate d'étain.

On peut aussi introduire l'étain (II) et le maintenir sous forme ionique dans la solution à partir d'autres sels d'étain, en particulier le chlorure d'étain, à condition d'ajouter simultanément à la solution un agent complexant ayant un pouvoir complexant vis-à-vis de l'étain, plus fort que ceux du ligand azoté et du composé (XVI) éventuellement utilisé dans la deuxième étape.

Dans ce cas, on ajoute du chlorure d'étain (II) et un agent complexant approprié à la solution du composé oxygéné de métal de transition et du ligand azoté.

A titre d'exemple d'agents complexants utilisables, on peut citer les pyrophosphates de métal alcalin ou d'ammonium, les glucoheptonates de métal alcalin ou d'ammonium, les diéthylène triamino pentacétates de métal alcalin ou d'ammonium, les éthylènediaminotétracétates de métal alcalin ou d'ammonium, les diamino-1,2 propane N,N,N',N'-tétracétates de métal alcalin ou d'ammonium, les gluconates de métal alcalin ou d'ammonium, les méthylènediphosphonates de métal alcalin ou d'ammonium, les hydroxyméthylènediphosphonates de métal alcalin ou d'ammonium et les citrates de métal alcalin ou d'ammonium.

Selon un second mode de réalisation de ce second procédé, l'agent réducteur est un dithionite d'ammonium ou d'un métal pharmaceutiquement acceptable.

Les dithionites de métaux pharmaceutiquement acceptables peuvent être en particulier les dithionites de métaux alcalins, par exemple le dithionite de sodium.

Comme on l'a vu précédemment, on réalise de préférence la réaction entre le composé oxygéné du métal de transition, le ligand azoté et l'agent réducteur dans une solution aqueuse dont le pH est ajusté à une valeur appropriée. On peut toutefois aussi opérer dans des solutions alcooliques ou hydroalcooliques.

Pour réaliser la réaction, on peut introduire aseptiquement une solution stérile du composé oxygéné de métal de transition et lui ajouter une solution stérile du ligand azoté et de l'agent réducteur dont le pH a été ajusté à la valeur voulue par addition d'acide, de base ou d'un tampon approprié. On peut ensuite effectuer la réaction à la température ambiante ou à une température supérieure, allant par exemple de 50 à 100°C, pendant des durées variables qui dépendent en particulier de la température utilisée.

Généralement, on opère avec des rapports molaires composé oxygéné du métal de transition / ligand azoté de 10⁻⁷ à 10⁻².

Après cette réaction, on peut ajouter le composé de formule (XVI), et le Laisser réagir à la température ambiante ou à une température supérieure pouvant aller par exemple de 37 à 45°C, dans le cas d'un anticorps monoclonal, pendant des durées variables qui dépendent en particulier de la température utilisée. Avec des ligands classiques on peut utiliser une température supérieure à la température ambiante pouvant aller par exemple de 50 à 100°C.

Généralement, on opère avec des rapports molaires, composé oxygéné du métal de transition / composé de formule (XVI) de 10⁻⁷ à 10⁻².

Dans cette deuxième étape, on peut aussi ajuster le pH de la solution à une valeur appropriée en introduisant le composé (XVI) dans une solution aqueuse ayant un pH adapté.

On peut aussi réaliser cette deuxième étape, en présence d'autres additifs, par exemple d'un agent complexant permettant d'éviter la réaction de l'étain avec le composé (XVI) utilisé, en particulier pour éviter la formation de précipités.

Comme précédemment, on réalise de préférence cette deuxième étape en solution aqueuse, mais on pourrait également la réaliser en solution alcoolique ou hydroalcoolique, ou encore effectuer la première étape et la deuxième étape dans des solutions différentes, par exemple la première étape en solution aqueuse et la deuxième étape en solution alcoolique ou hydroalcoolique ou l'inverse.

Lorsque le produit radiopharmaceutique de l'invention est destiné au diagnostic, il est généralement nécessaire dé le préparer au moment de l'utilisation.

Aussi, l'invention a également pour objet une trousse pour la préparation d'un produit radiopharmaceutique à tropisme cardiaque, qui comprend :
- un premier flacon contenant une phosphine ou un agent réducteur choisi parmi le dithionite d'ammonium, les dithionites de métaux pharmaceutiquement acceptables et l'étain (II) sous forme ionique ;
- un second flacon contenant de l'azoture d'ammonium, de l'azoture d'un métal pharmaceutiquement acceptable, ou un ligand azoté, et
- un troisième flacon contenant un composé répondant à la formule : 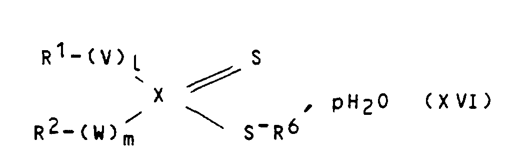 dans laquelle R¹, R², V, W, l, m, n et X ont la signification donnée ci-dessus, R⁶ est un ion de métal alcalin, H⁺ ou NH₄⁺, et p est égal à 0 ou est un nombre entier allant de 1 à 5.

Avec cette trousse, on peut préparer directement le produit radiopharmaceutique voulu, dans un service hospitalier de médecine nucléaire, en mélangeant le contenu des deux premiers flacons avec une solution du composé oxygéné de métal de transition, par exemple une solution de pertechnétate de métal alcalin ou d'ammonium, puis ajouter au produit ainsi obtenu le contenu du troisième flacon.

Les produits présents respectivement dans les premier, second et troisième flacons peuvent être sous forme liquide ou sous forme lyophilisée.

Dans certains cas, on peut aussi mélanger le contenu des deux premiers flacons avant utilisation. Dans ce cas, la trousse comprendra uniquement un premier flacon contenant une phosphine ou l'agent réducteur mélangé au second réactif constitué soit par l'azoture, soit par l'acide dithiocarbazique ou un dérivé de celui-ci, et un second flacon contenant le composé de formule (XVI) donnée ci-dessus.

Etant donné que les produits sont destinés à être administrés, par exemple par voie intraveineuse à des êtres vivants, on doit utiliser des conditions appropriées de fabrication et de mise en oeuvre pour obtenir des solutions Convenablement stériles et apyrogènes.

Pour préparer les solutions, on peut utiliser de l'eau stérile et apyrogène ou des solutions alcooliques ou hydroalcooliques, stériles et apyrogènes, et effectuer un stockage des solutions sous azote.

Pour préparer des compositions lyophilisées, on soumet à une lyophilisation dans un équipement classique des solutions obtenues dans les mêmes conditions que précédemment.

Les produits radiopharmaceutiques de l'invention peuvent être utilisés en particulier pour la scintigraphie du cerveau.

Dans ce cas, après préparation du complexe nitruro de technétium, on injecte celui-ci au patient à examiner, et on procède ensuite à un examen du cerveau par scintigraphie.

Pour l'injection du produit, les quantités des différents réactifs sont telles qu'elles correspondent sensiblement à la stoechiométrie des complexes à obtenir. La quantité finale injectée dépend en particulier des réactifs utilisés et de leur toxicité.

Généralement, on obtient des résultats satisfaisants en utilisant des quantités totales de réactifs allant de 0,05 à 0,40mg/kg de poids corporel.

La dose totale de métal de transition par exemple de technétium, se situe généralement dans la gamme de 185 à 740Mbq (5 à 20millicuries).

Après l'administration du complexe nitruro de métal de transition, on peut effectuer un examen satisfaisant dans un délai de 0,5 à 3h en obtenant un bon contraste, des images nettes et une bonne détection des lésions.

D'autres caractéristiques et avantages de l'invention apparaîtront mieux à la lecture des exemples suivants, donnés bien entendu à titre illustratif et non limitatif.

### Exemple 1: Préparation du complexe nitruro-bis (diméthyldithiophosphinato)^{99m}Tc(V)(TcNDMDP).

### a) Préparation du produit intermédiaire.

Dans un flacon type pénicilline, on introduit 0,4ml d'une solution contenant 2.10⁻²mol/l (2,5mg/ml)de S-méthyldithiocarbazate dans l'alcool éthylique, puis 0,2ml d'une solution à 2.10⁻²mol/l (5mg/ml)de triphénylphosphine dans l'alcool éthylique et 0,1ml d'acide chlorhydrique 1N. On ajoute ensuite 0,5 à 1ml d'une solution de pertechnétate de sodium (Tc^{99m}) et on effectue la réaction à 80°C pendant 30 minutes ou à 100°C pendant 15 minutes.

### b) Préparation du complexe final.

Au contenu du flacon obtenu dans l'étape a), on ajoute 1ml d'une solution tampon d'acétate de sodium 0,5M pH 5,0 et 0,5ml d'une solution 6,7.10⁻²M, de diméthyldithiophosphinate de sodium dans le tampon acétate 0,2M pH 5,0.

On effectue la réaction pendant 30 minutes à la température ambiante.

La pureté radiochimique du complexe obtenu est testée en effectuant une chromatographie sur couche mince en utilisant un gel de silice et un mélange 1:1 de dichlorométhane et de toluène comme solvant.

Le complexe obtenu a un R_{f} de 0,53±0,03.

Si la pureté radiochimique est inférieure à 80%, le complexe est purifié par chromatographie sur couche mince préparative. Dans ce cas, après développement du solvant, on extrait par l'alcool éthylique la radioactivité correspondante à l'endroit de migration du produit pur. La pureté radiochimique du produit final après cette purification est supérieure ou égale à 93%.

### Exemple 2 : Préparation du complexe nitruro-bis(diéthyldithiophosphinato)^{99m}Tc(V) (TcNDEDP).

### a) Préparation du produit intermédiaire.

Dans un flacon type pénicilline, on introduit 0,2ml d'une solution contenant 7,7.10⁻²mol/l (5,0mg/ml) d'azoture de sodium dans de l'eau, puis 0,2ml d'une solution à 2.10⁻²mol/l (5mg/ml) de triphénylphosphine dans l'alcool éthylique et 0,1ml d'acide chlorhydrique 1N. On ajoute ensuite 0,5 à 1ml d'une solution de pertechnétate de sodium (Tc-99m) et on effectue la réaction à 80°C pendant 30 minutes ou à 100°C pendant 15 minutes.

### b) Préparation du produit final.

Au contenu du flacon obtenu dans l'étape a), on ajoute 1ml d'une solution tampon acétate 0,5M, pH 5,0 et 0,5ml d'une solution 6.10⁻²M de diéthyl dithiophosphinate de sodium dans le tampon acétate 0,2M, pH 5,0.

On effectue la réaction pendant 30 minutes à la température ambiante.

La pureté radiochimique du complexe obtenu est testée en effectuant une chromatographie sur couche mince en utilisant un gel de silice et un mélange 1:1 de dichlorométhane et le toluène comme solvant.

Le complexe obtenu a un R_{f} de 0,80±0,03.

La pureté radiochimique est supérieure ou égale à 90%.

### Exemple 3 : Préparation du complexe nitruro(diméthyldithiophosphinato, diéthyldithiophosphinato) ^{99m}Tc(V) (TcNMEDP).

### a) Préparation du produit intermédiaire.

Dans un flacon du type pénicilline, on introduit :
- 0,5 à 3ml d'une solution stérile de pertechnétate de sodium (technétium-99m) correspondant à une radioactivité allant de 18MBq à 3,7GBq (0,5 à 100mCi),
- 1ml d'un tampon phosphate ayant une concentration molaire allant de 0,1 à 0,5M et un pH de 7,4 à 8,
- 0,1 à 0,5ml d'une solution aqueuse contenant 2.10⁻²mol/l (2,7mg/ml) de S-méthyl, N-méthyl dithiocarbazate, et
- 0,1 à 0,3ml d'une solution aqueuse contenant 1,8.10⁻³mol/l de chlorure d'étain (II) dihydraté et 5,6.10⁻² mol/l de pyrophosphate de sodium.

On effectue la réaction à la température ambiante pendant 30min.

### b) Préparation du produit final.

Au contenu du flacon obtenu dans l'étape a), on ajoute 1ml d'une solution de tampon acétate 0,5M, pH 5,0 puis 0,5ml d'une solution 6,7.10⁻²M, de diméthyl dithiophosphinate de sodium dans le tampon acétate 0,2M, pH 5,0 et 0,3ml d'une solution 6.10⁻²M de diéthyldithiophosphinate dans le tampon acétate 0,2M pH 5,0.

On effectue la réaction pendant 30 minutes à température ambiante.

Les conditions de réaction conduisent à la formation de trois produits : le nitruro bis (diméthyldithiophosphinato)^{99m}Tc V, le nitruro bis (diéthyldithiophosphinato)^{99m}Tc V et le nitruro(diméthyldithiophosphinato, diéthyldithiophosphinato)^{99m}Tc V, ce dernier étant statistiquement favorisé. Ce produit est séparé par chromatographie sur couche mince préparative sur gel de silice en utilisant du dichlorométhane et du toluène (1:1) comme solvant. On repère les emplacements des deux complexes symétriques au moyen d'un échantillon témoin et on déduit l'emplacement du complexe mixte.

Celui-ci est extrait du gel de silice par lavage à l'alcool éthylique. La pureté du produit final après purification par chromatographie sur couche mince (CCM) est supérieure à 90%.

### Exemple 4 : Préparation du complexe nitruro bis (N-(N,N diméthyléthylamino), N-méthyl dithiocarbamato)^{99m}Tc(V)(TcN-NDMMDC).

### a) Préparation du produit intermédiaire.

Dans un flacon type pénicilline, on introduit 0,2ml d'une solution contenant 7,7.10⁻²mol/l (5mg/ml) d'azoture de sodium dans l'eau, puis 0,4ml d'une solution contenant 1,10⁻²mol/l de tris(2-cyanoéthyl)phosphine.

On ajoute ensuite 0,5 à 5ml d'une solution de pertechnétate de sodium (^{99m}Tc) et on effectue la réaction à 80°C pendant 30 minutes ou à 100°C pendant 15 minutes.

Cette opération a lieu à un pH proche de 7.

### b) Préparation du produit final.

Au contenu du flacon obtenu dans l'étape a), on ajoute 0,1ml d'une solution de NaOH 1N et 0,5ml d'une solution de N-(N,N diméthyléthylamino,
N-méthyldithiocarbamate de sodium 0,1M dans une solution tampon phosphate 0,2M, pH 7,6.

On effectue la réaction à la température ambiante pendant 30 minutes. La pureté radiochimique du complexe obtenu est testée en effectuant une chromatographie sur couche mince en utilisant un gel de silice et un solvant constitué d'un mélange d'éthanol, de chloroforme, de toluène et d'acétate d'ammonium 0,1M dans les rapports volumiques 6:3:3:1. Le R_{f} du produit est 0,60±0,03. La pureté radiochimique est supérieure ou égale à 93%.

### Exemple 5 : Préparation du complexe nitruro bis (N-(N,N diéthyléthylamino), N-méthyl dithiocarbamato)^{99m}Tc(V).

On suit le même mode opératoire que dans l'exemple 1 pour préparer le produit intermédiaire.

Pour la préparation du produit final, on suit le mode opératoire de l'exemple 4 en utilisant une solution à 0,1mol/l de N-(N,N diéthyléthylamino), N-méthyl dithiocarbamate de sodium, dans une solution tampon phosphate 0,2M à pH 7,6.

La pureté radiochimique du complexe obtenu est testée en effectuant une chromatographie sur couche mince en utilisant un gel de silice et un solvant constitué d'un mélange d'éthanol, de chloroforme, de toluène, et d'acétate d'ammonium 0,1M dans les rapports volumiques 6:3:3:1. Le R_{f} du produit est 0,80±0,02.

La pureté radiochimique est supérieure ou égale à 93%.

### Exemple 6 : Préparation du complexe nitruro bis (N-méthylène méthyl carboxylate, N-méthyl dithiocarbamato)^{99m}Tc (V).

On prépare le produit intermédiaire en suivant le même mode opératoire que dans l'exemple 3, puis on prépare le produit final de la façon suivante.

Au contenu du flacon obtenu dans l'étape de préparation du produit intermédiaire, on ajoute 0,1ml d'une solution de N-méthylène carboxylate, N-méthyl dithiocarbamate de sodium à 0,5mol/l dans l'eau.

Le schéma réactionnel est le suivant :

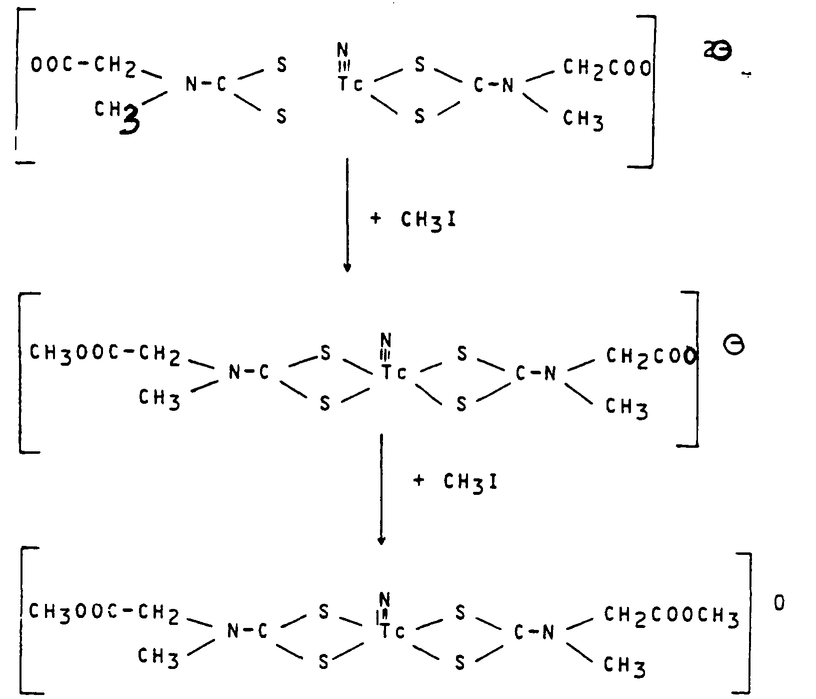

La réaction conduisant au nitruro bis (N-méthylène méthyl carboxylate, N-méthyl dithiocarbamato)^{99m}Tc (V) (1) a lieu en 10 minutes à la température ambiante.

A 0,25ml de cette solution, on ajoute 1,5ml de diméthylsulfoxyde (DMSO) et 0,2ml (3,2.10⁻³mol) d'iodure de méthyle. La réaction d'estérification du (ou des) carboxyles libres a lieu pendant 30 minutes à la température ambiante.

On évapore l'excès d'iodure de méthyle sous courant d'azote, puis on ajoute 1,5ml d'une solution de tampon acétate 0,2M, pH 5,0 et 1,5ml d'éthanol. Le mélange est passé sur une colonne contenant 2ml d'une résine échangeuse d'ion à matrice de polystyrène comportant des groupements aminoquaternaires capables de fixer les composés ayant une ou plusieurs charges négatives. On sépare ainsi le produit final (3), qui est neutre, du produit de départ (1) et du sous-produit (2) monoester qui sont, par contre, chargés négativement.

La pureté radiochimique est testée par chromatographie sur couche mince sur gel de silice en utilisant un solvant constitué d'un mélange d'éthanol, de chloroforme, de toluène et d'acétate d'ammonium 0,1M dans les rapports volumiques 6:3:3:1. Le R_{f} du produit final est 0,88±0,02. La pureté radiochimique est supérieure ou égale à 95%.

### Exemple 7.

On teste les propriétés biologiques des complexes obtenus dans les exemples 1 à 5 en déterminant leur biodistribution chez des rats mâles de la race Sprague Dawley pesant 200±20g.

Dans ce cas, on injecte aux rats, anesthésiés avec du pentobarbital de sodium, une dose correspondant à 15µmol/kg de masse corporelle de complexe, ce qui correspond à une dose de radioactivité de 1 à 2,5µCi.

5 minutes, 30 minutes, ou 60 minutes après l'injection du produit, on sacrifie les rats, on prélève leurs organes et on détermine la radioactivité présente dans chacun des organes.

les résultats obtenus sont donnés dans le tableau 1 qui suit, et exprimés en pourcentages de la radioactivité injectée retrouvée dans l'organe, après prélèvement et comptage.

Les valeurs données dans chaque case du tableau représentent la valeur moyenne et les deux valeurs extrêmes.

On reporte aussi le rapport entre les pourcentages de la radioactivité injectée retrouvée par gramme de cerveau et par gramme de sang qui est un indice de la qualité du contraste.

### Exemple 8.

Dans cet exemple, on teste les propriétés biologiques des complexes obtenus dans les exemples 4 et 6 en déterminant leur captation par le cerveau chez des singes Macaques Cynomolgus pesant entre 7,5 et 8,5kg.

Dans ce cas, on injecte, aux singes anesthésiés à la kétamine, une dose correspondant à 1,25µmol/kg du produit décrit dans l'exemple 4 et 0,30µmol/kg du produit décrit dans l'exemple 6, ce qui correspond à une dose de radioactivité de 4 à 6mCi.

La captation de la radioactivité par le cerveau et les organes ou tissus environnants (coeur, poumons, muscles, thyroide, tête) est déterminée par acquisition dynamique, entre l'injection et la fin de l'examen, à la gamma-caméra, en définissant des zones d'intérêt pour chaque organe.

Pendant l'acquisition, l'animal est positionné en décubitus latéral droit.

Les valeurs sont exprimées en activité par pixel (unité de surface) par minute et par mCi ; elles ne sont pas corrigées par la décroissance radioactive du radioélément.

Les résultats obtenus sont donnés dans le tableau 2 qui suit.

On remarque un meilleur rapport cerveau/muscle pour le complexe de l'exemple 4 et une très bonne fixation cérébrale pour le complexe de l'exemple 6.

Les produits radiopharmaceutiques de l'invention sont donc très intéressants comme produit de diagnostic ou de thérapie pour le cerveau.

### Exemple 9 : Préparation du complexe nitruro bis (N-méthylène éthyl carboxylate, N-méthyl dithiocarbamato)^{99m}Tc (V).

On prépare le produit intermédiaire en suivant le même mode opératoire que dans l'exemple 3, puis on prépare le produit final de la façon suivante.

Au contenu du flacon obtenu dans l'étape de préparation du produit intermédiaire, on ajoute 0,1ml d'une solution de N-méthylène carboxylate, N-méthyl dithiocarbamate de sodium à 0,5mol/l dans l'eau.

La réaction conduisant au nitruro bis (N-méthylène carboxylate, N-méthyl dithiocarbamato) ^{99m}Tc (V) a lieu en 10 minutes à température ambiante.

A 0,25ml de cette solution, on ajoute 1,5ml de diméthyl sulfoxyde (DMSO) et 0,2ml (2,5x10⁻³ mol) d'iodure d'éthyle. La réaction d'estérification des carboxyles libres a lieu en 30 minutes à température ambiante. On évapore l'excès d'iodure d'éthyle sous courant d'azote, puis on ajoute 1,5ml d'une solution de tampon acétate 0,2M - pH=5 et 1,5ml d'éthanol. Le mélange est passé sur une colonne contenant 2ml d'une résine échangeuse d'anions. On sépare ainsi le produit final qui est neutre, du produit de départ et du sous produit monoester qui sont par contre chargés négativement.

La pureté radiochimique est testée par chromatographie sur couche mince sur gel de silice en utilisant un solvant constitué d'un mélange d'éthanol, de chloroforme, de toluène et d'acétate d'ammonium 0,1M dans l'eau dans les rapports volumiques 6:3:3:1. Le R_{f} du produit final est 0,90± 0,02. La pureté radiochimique est supérieure ou égale à 80%.

### Exemple 10 : Préparation du complexe nitruro bis (N-méthylène méthyl carboxylate, N-éthyl dithiocarbamato)^{99m}Tc (V).

On prépare le produit intermédiaire en suivant le même mode opératoire que dans l'exemple 3, puis on prépare le produit final de la façon suivante.

Au contenu du flacon obtenu dans l'étape de préparation du produit intermédiaire, on ajoute 0,1ml d'une solution de N-méthylène carboxylate, N-éthyl dithiocarbamate de sodium à 0,5mol/l dans l'eau.

La réaction conduisant au nitruro bis(N-méthylène carboxylate, N-éthyl dithiocarbamato)^{99m}Tc (V) a lieu en 10 minutes à température ambiante.

A 0,25ml de cette solution, on ajoute 1,5ml de diméthyl sulfoxyde (DMSO) et 0,2ml (3,2 x 10⁻³ mol) d'iodure de méthyle. La réaction d'estérification des carboxyles libres a lieu en 30 minutes à température ambiante. On évapore l'excès d'iodure de méthyle sous courant d'azote, puis on ajoute 1,5ml d'une solution de tampon acétate 0,2M, pH=5 et 1,5ml d'éthanol. Le mélange est passé sur une colonne contenant 2ml d'une résine échangeuse d'anions. On sépare ainsi le produit final qui est neutre, du produit de départ et du sous-produit monoester qui sont par contre chargés négativement.

La pureté radiochimique est testée par chromatographie sur couche mince sur gel de silice en utilisant un solvant constitué d'un mélange d'éthanol, de chloroforme, de toluène et d'acétate d'ammonium 0,1M dans l'eau dans les rapports volumiques 6:3:3:1. Le Rf du produit final est 0,90± 0,02. La pureté radiochimique est supérieure ou égale à 92%.

### Exemple 11 : Préparation du complexe nitruro bis(N-méthylène méthyl carboxylate, N-propyl dithiocarbamato)^{99m}Tc (V).

On prépare le produit intermédiaire en suivant le même mode opératoire que dans l'exemple 3, puis on prépare le produit final de la façon suivante.

Au contenu du flacon obtenu dans l'étape de préparation du produit intermédiaire, on ajoute 0,1ml d'une solution de N-méthylène carboxylate, N-propyl dithiocarbamate de sodium à 0,5mol/l dans l'eau.

La réaction conduisant au nitruro bis(N-méthylène carboxylate, N-propyl dithiocarbamato)^{99m}Tc (V) a lieu en 10 minutes à température ambiante.

A 0,25ml de cette solution, on ajoute 1,5ml de diméthyl sulfoxyde (DMSO) et 0,2ml (3,2x10⁻³ mol) d'iodure de méthyle. La réaction d'estérification des carboxyles libres a lieu en 30 minutes à température ambiante. On évapore l'excès d'iodure de méthyle sous courant d'azote, puis on ajoute 1,5ml d'une solution de tampon acétate 0,2M, pH=5 et 1,5ml d'éthanol. Le mélange est passé sur une colonne contenant 2ml d'une résine échangeuse d'anions. On sépare ainsi le produit final qui est neutre, du produit de départ et du sous produit monoester qui sont par contre chargés négativement.

La pureté radiochimique est testée par chromatographie sur couche mince sur gel de silice en utilisant un solvant constitué d'un mélange d'éthanol, de chloroforme, de toluène et d'acétate d'ammonium 0,1M dans l'eau dans les rapports volumiques 6:3:3:1. Le Rf du produit final est 0,92± 0,02. La pureté radiochimique est supérieure ou égale à 95%.

### Exemple 12 : Préparation du complexe nitruro bis (N-méthylène éthyl carboxylate, N-n-propyl dithiocarbamato)^{99m}Tc (V).

On prépare le produit intermédiaire en suivant le même mode opératoire que dans l'exemple 3, puis on prépare le produit final de la façon suivante.

Au contenu du flacon obtenu dans l'étape de préparation du produit intermédiaire, on ajoute 0,1ml d'une solution de N-méthylène carboxylate, N-propyl dithiocarbamate de sodium à 0,5mol/l dans l'eau.

La réaction conduisant au nitruro bis(N-méthylène carboxylate, N-propyl dithiocarbamato)^{99m}Tc (V) a lieu en 10 minutes à température ambiante.

A 0,25ml de cette solution, on ajoute 1,5ml de diméthyl sulfoxyde (DMSO) et 0,2ml (2,5x10⁻³ mol) d'iodure d'éthyle. La réaction d'estérification des carboxyles libres a lieu en 30 minutes à température ambiante. On évapore l'excès d'iodure d'éthyle sous courant d'azote, puis on ajoute 1,5ml d'une solution de tampon acétate 0,2M - pH=5 et 1,5ml d'éthanol. Le mélange est passé sur une colonne contenant 2ml d'une résine échangeuse d'anions. On sépare ainsi le produit final qui est neutre, du produit de départ et du sous produit monoester qui sont par contre chargés négativement.

La pureté radiochimique est testée par chromatographie sur couche mince sur gel de silice en utilisant un solvant constitué d'un mélange d'éthanol, de chloroforme, de toluène et d'acétate d'ammonium 0,1M dans l'eau dans les rapports volumiques 6:3:3:1. Le Rf du produit final est 0,92± 0,02. La pureté radiochimique est supérieure ou égale à 95%.

### Exemple 13 :Préparation du complexe nitruro bis (3-(méthyl carboxylate)pipéridino dithiocarbamato)^{99m}Tc (V).

On prépare le produit intermédiaire en suivant le même mode opératoire que dans l'exemple 3, puis on prépare le produit final de la façon suivante.

Au contenu du flacon obtenu dans l'étape de préparation du produit intermédiaire, on ajoute 0,1ml d'une solution de (3-carboxylate) pipéridino dithiocarbamate de sodium à 0,5mol/l dans l'eau.

La réaction conduisant au nitruro bis ((3-carboxylate)pipéridino dithiocarbamato)^{99m}Tc (V) a lieu en 10 minutes à température ambiante.

A 0,25ml de cette solution, on ajoute 1,5ml de diméthyl sulfoxyde (DMSO) et 0,2ml (3,2x10⁻³ mol) d'iodure de méthyle. La réaction d'estérification des carboxyles libres a lieu en 30 minutes à température ambiante. On évapore l'excès d'iodure de méthyle sous courant d'azote, puis on ajoute 1,5ml d'une solution de tampon acétate 0,2M, pH=5 et 1,5ml d'éthanol. Le mélange est passé sur une colonne contenant 2ml d'une résine échangeuse d'anions. On sépare ainsi le produit final qui est neutre, du produit de départ et du sous produit monoester qui sont par contre chargés négativement.

La pureté radiochimique est testée par chromatographie sur couche mince sur gel de silice en utilisant un solvant constitué d'un mélange d'éthanol, de chloroforme, de toluène et d'acétate d'ammonium 0,1M dans l'eau dans les rapports volumiques 6:3:3:1. Le Rf du produit final est 0,94± 0,02. La pureté radiochimique est supérieure ou égale à 95%.

### Exemple 14 : Préparation du complexe nitruro bis [bis N-(méthylène méthyl carboxylate)] dithiocarbamato)^{99m}Tc (V).

On prépare le produit intermédiaire en suivant le même mode opératoire que dans l'exemple 3, puis on prépare le produit final de la façon suivante.

Au contenu du flacon obtenu dans l'étape de préparation du produit intermédiaire, on ajoute 0,1ml d'une solution de bis(méthylène carboxylate) dithiocarbamate de sodium à 0,5mol/l dans l'eau.

La réaction conduisant au nitruro bis (bis(méthylène carboxylate) dithiocarbamato)^{99m}Tc (V) a lieu en 10 minutes à température ambiante.

A 0,25ml de cette solution, on ajoute 1,5ml de diméthyl sulfoxyde (DMSO) et 0,2ml (3,2x10⁻³ mol) d'iodure de méthyle. La réaction d'estérification des carboxyles libres a lieu en 30 minutes à température ambiante. On évapore l'excès d'iodure de méthyle sous courant d'azote, puis on ajoute 1,5ml d'une solution de tampon acétate 0,2M, pH=5 et 1,5ml d'éthanol. Le mélange est passé sur une colonne contenant 2ml d'une résine échangeuse d'anions. On sépare ainsi le produit final qui est neutre, du produit de départ et des sous produits monoester, diester et triester qui sont par contre chargés négativement.

La pureté radiochimique est testée par chromatographie sur couche mince sur gel de silice en utilisant un solvant constitué d'un mélange d'éthanol, de chloroforme, de toluène et d'acétate d'ammonium 0,1M dans l'eau dans les rapports volumiques 6:3:3:1. Le Rf du produit final est 0,86± 0,02. La pureté radiochimique est supérieure ou égale à 85%.

### Exemple 15 :Préparation du complexe nitruro bis (N-éthylène-2(méthyl carboxylate), N-méthyl dithiocarbamato)^{99m}Tc (V).

On prépare le produit intermédiaire en suivant le même mode opératoire que dans l'exemple 3, puis on prépare le produit final de la façon suivante.

Au contenu du flacon obtenu dans l'étape de préparation du produit intermédiaire, on ajoute 0,1ml d'une solution de N-éthylène (2-carboxylate), N-méthyl dithiocarbamate de sodium à 0,5mol/l dans l'eau.

La réaction conduisant au nitruro bis (N-éthylène(2-carboxylate), N-méthyl dithiocarbamato)^{99m}Tc (V) a lieu en 10 minutes à température ambiante.

A 0,25ml de cette solution, on ajoute 1,5ml de diméthyl sulfoxyde (DMSO) et 0,2ml (3,2 x 10⁻³mol) d'iodure de méthyle. La réaction d'estérification des carboxyles libres a lieu en 30 minutes à température ambiante. On évapore l'excès d'iodure de méthyle sous courant d'azote, puis on ajoute 1,5ml d'une solution de tampon acétate 0,2M, pH=5 et 1,5ml d'éthanol. Le mélange est passé sur une colonne contenant 2ml d'une résine échangeuse d'anions. On sépare ainsi le produit final qui est neutre, du produit de départ et du sous produit monoester qui sont par contre chargés négativement.

La pureté radiochimique est testée par chromatographie sur couche mince sur gel de silice en utilisant un solvant constitué d'un mélange d'éthanol, de chloroforme, de toluène et d'acétate d'ammonium 0,1M dans l'eau dans les rapports volumiques 6:3:3:1. Le Rf du produit final est 0,90± 0,02. La pureté radiochimique est supérieure ou égale à 95%.

### Exemple 16.

### a) Préparation du ligand N-méthylène méthyl carboxylate, N-méthyl dithiocarbamate de sodium.

Le ligand est préparé à partir du chlorhydrate de l'ester méthylique de la sarcosine suivant le schéma réactionnel suivant :

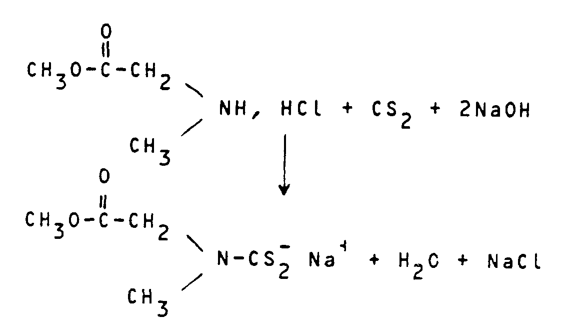

Une solution contenant 8g de NaOH (0,2 mole) dans 200ml de méthanol est refroidie à -10°C. On ajoute à cette solution 14,2g de sarcosine méthyl ester chlorhydrate (0,1 mole) puis 7,6g de CS₂ (0,1 mole). On Laisse réagir 30 minutes en maintenant la solution à -10°C. Le produit est ensuite séparé sur une colonne de silice en utilisant comme éluant du méthanol. Les fractions correspondant au produit final sont regroupées et le solvant évaporé. On obtient une huile jaune - marron.

Le ligand est caractérisé par RMN du proton dans le DMSO :

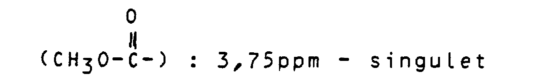

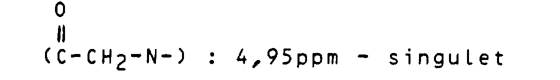

(CH₃-N-) : 3,70ppm - singulet.

### b) Préparation du nitruro bis (N-méthylène méthyl carboxylate, N-méthyl dithiocarbamato)^{99m}Tc.

On prépare le produit intermédiaire en suivant le même mode opératoire que dans l'exemple 3, puis on prépare le produit final de la façon suivante.

Au contenu du flacon obtenu dans l'étape de préparation du produit intermédiaire, on ajoute 3ml d'une solution de N-méthylène méthyl carboxylate, N-méthyl dithiocarbamate de sodium à 0,05mol/l dans l'eau. La réaction conduisant au nitruro bis (N-méthylène méthyl carboxylate N-méthyl dithiocarbamato)^{99m}Tc (V) a lieu en 10 minutes à température ambiante.

La pureté radiochimique est testée par chromatographie sur couche mince sur gel de silice en utilisant un solvant constitué d'un mélange d'éthanol, de chloroforme, de toluène et d'acétate d'ammonium 0,1M dans l'eau dans les rapports volumiques 6 : 3 :3 : 1. Le Rf du produit final est 0,88±0,02. La pureté radiochimique est supérieure ou égale à 90%.

On obtient ainsi le même complexe que dans l'exemple 6.

Les formules des complexes obtenus dans les exemples 9 à 16 sont données dans le tableau 3 qui suit.

### Exemple 17.

Dans cet exemple, on teste les propriétés déterminant leur captation par le cerveau chez le singe, selon le même mode opératoire que dans l'exemple 8.

Les valeurs de fixation sont exprimées en coups par pixel, par mCi et par minute, et elles sont reportées dans le tableau 4 qui suit.

Au vu de ces résultats, on remarque que les complexes des exemples 9, 12 et 14 ont une fixation cérébrale moyenne et que tous les autres complexes ont une bonne fixation cérébrale.

Par ailleurs, les images obtenues sont de très bonne qualité, ce qui montre l'intérêt des produits radiopharmaceutiques de l'invention pour l'imagerie ou la thérapie du cerveau.

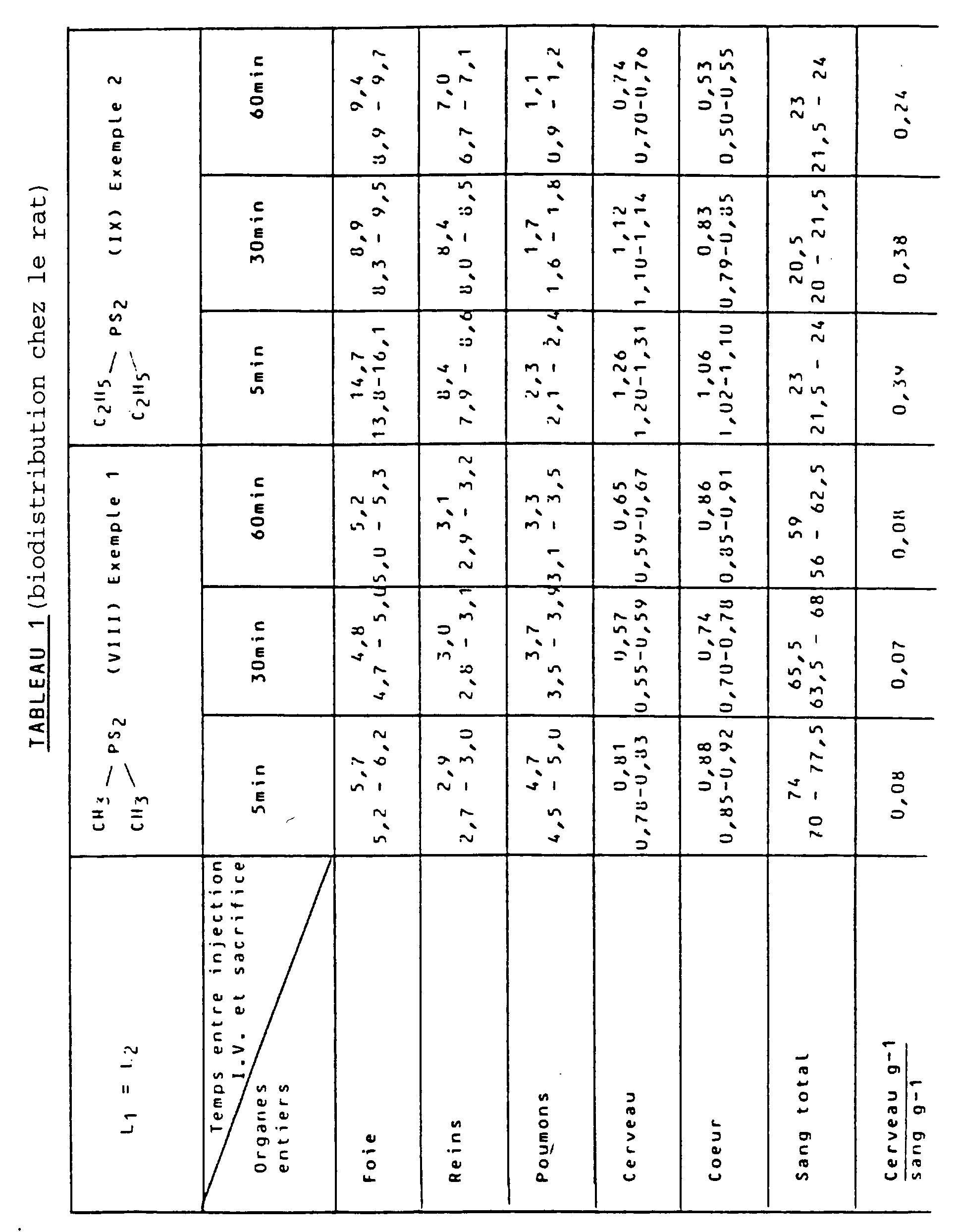

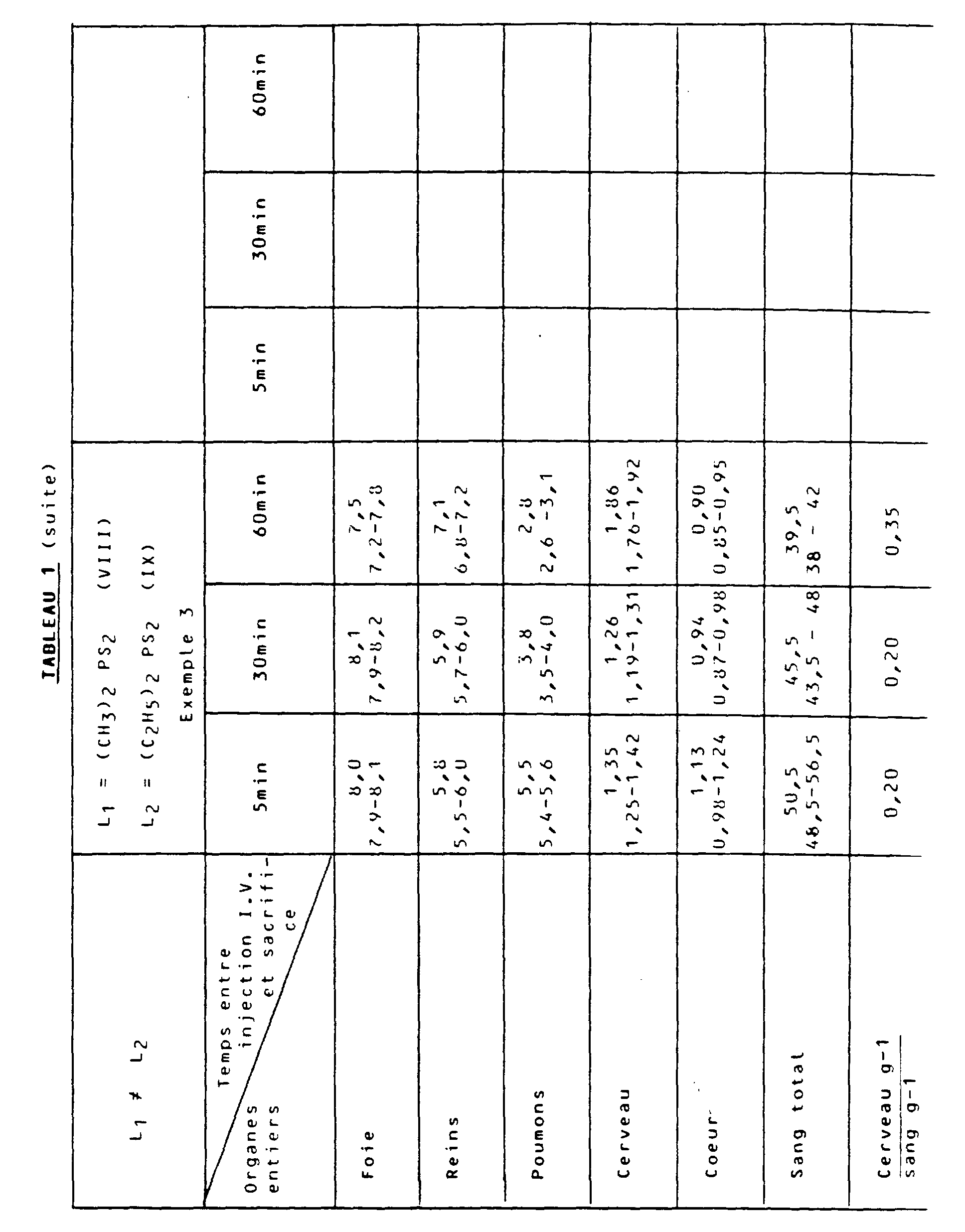

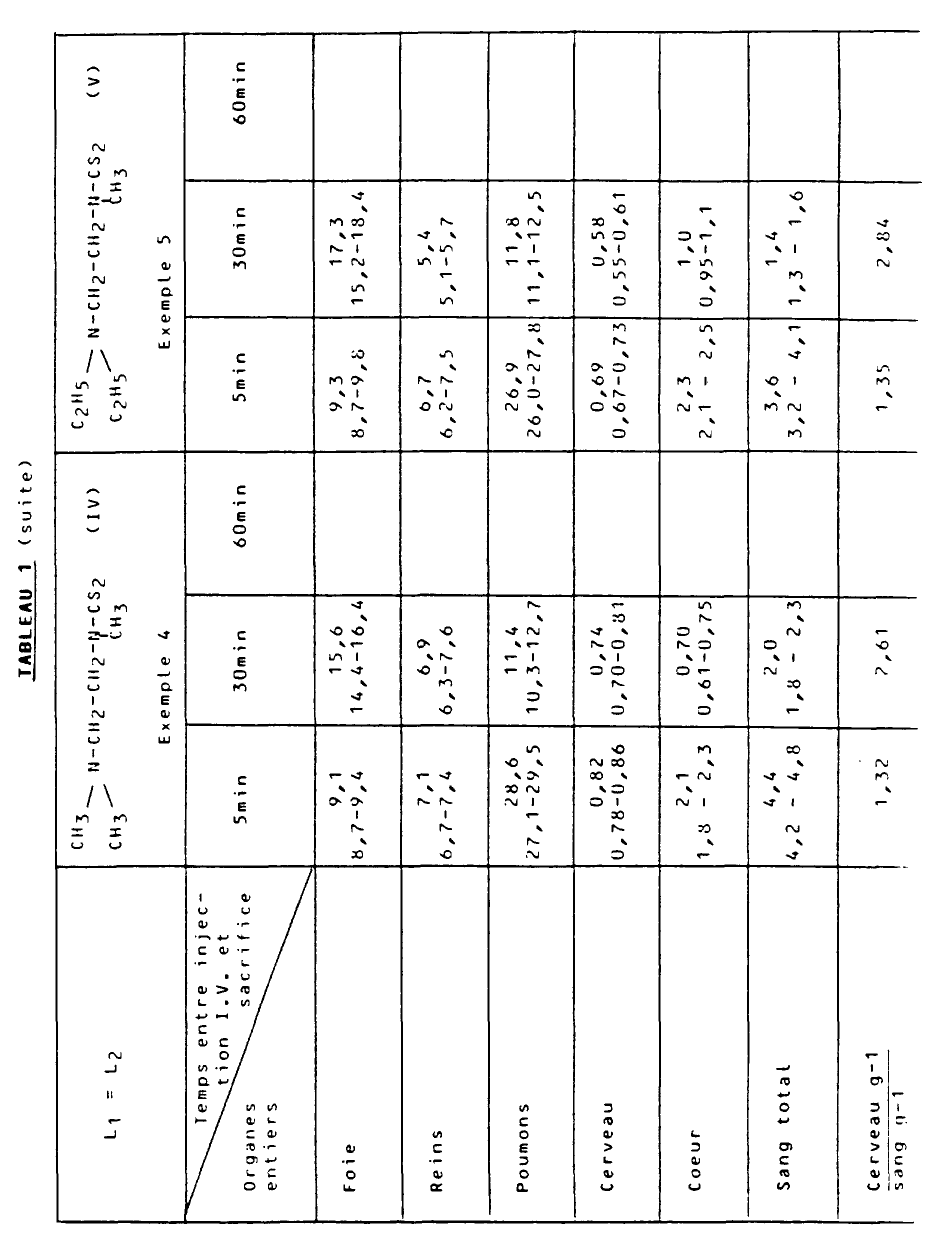

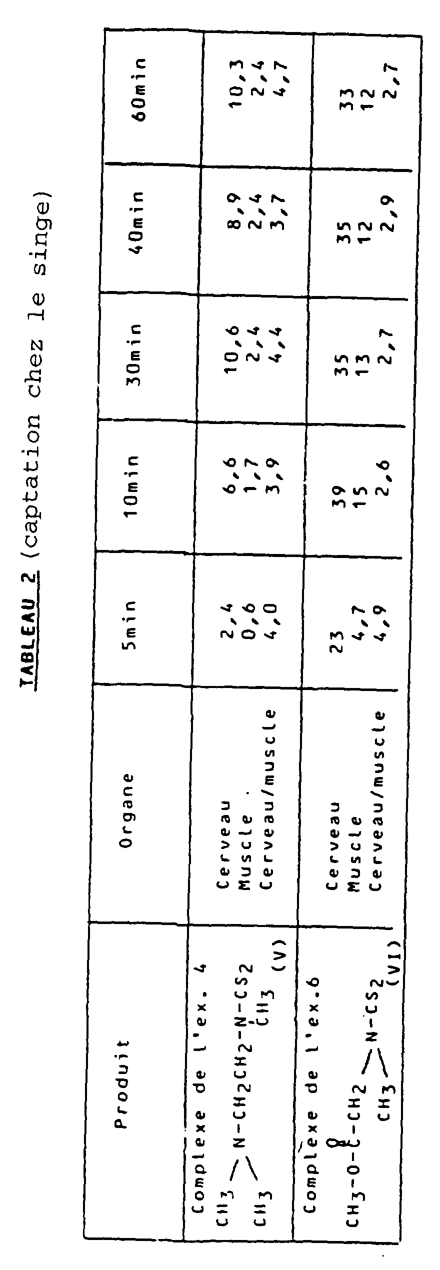

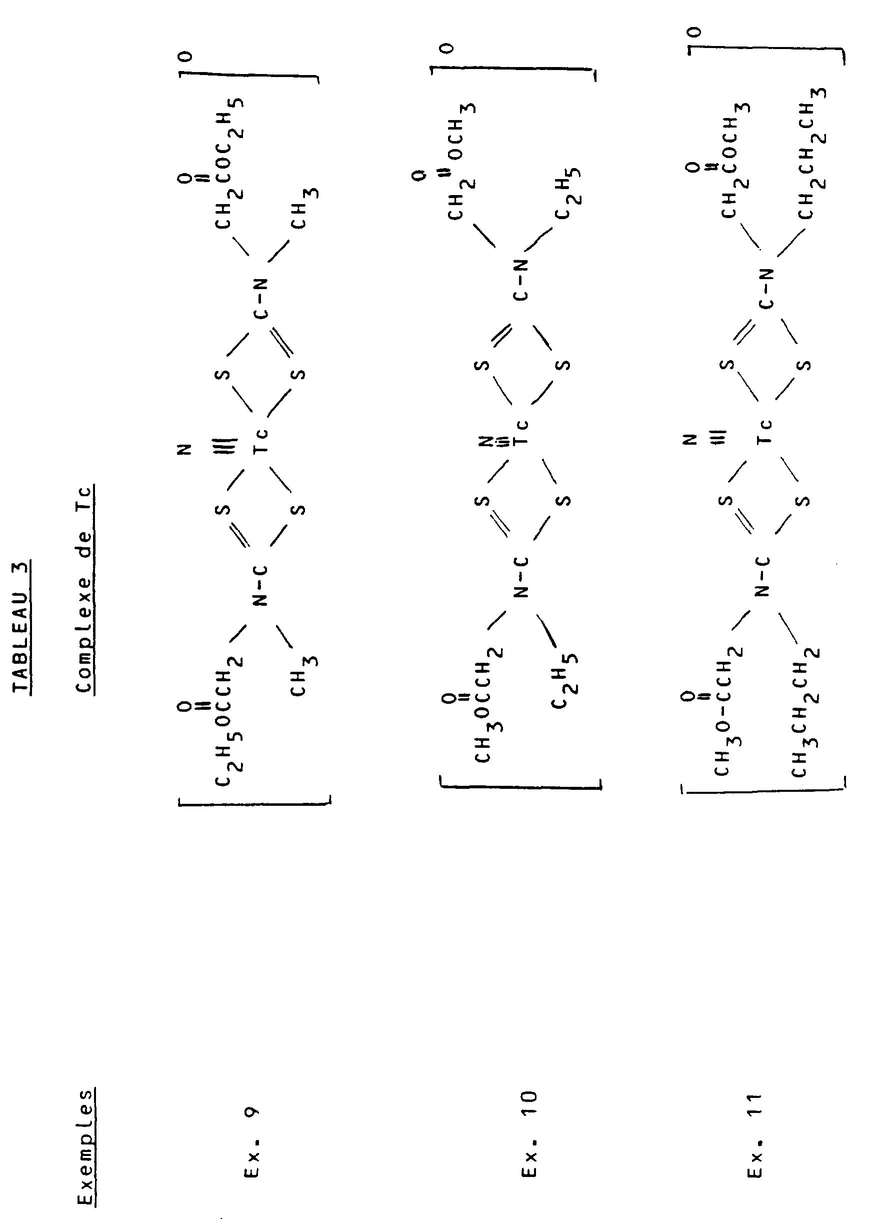

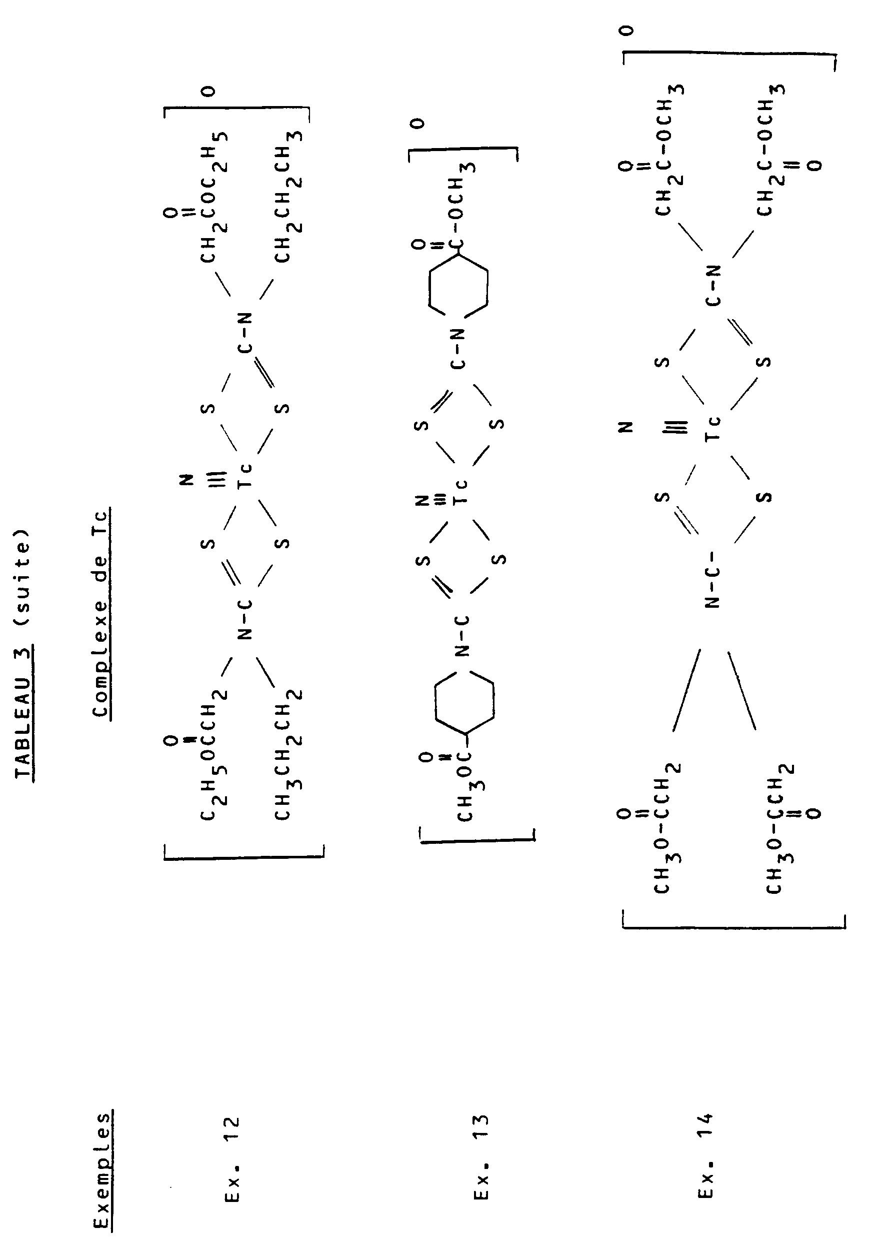

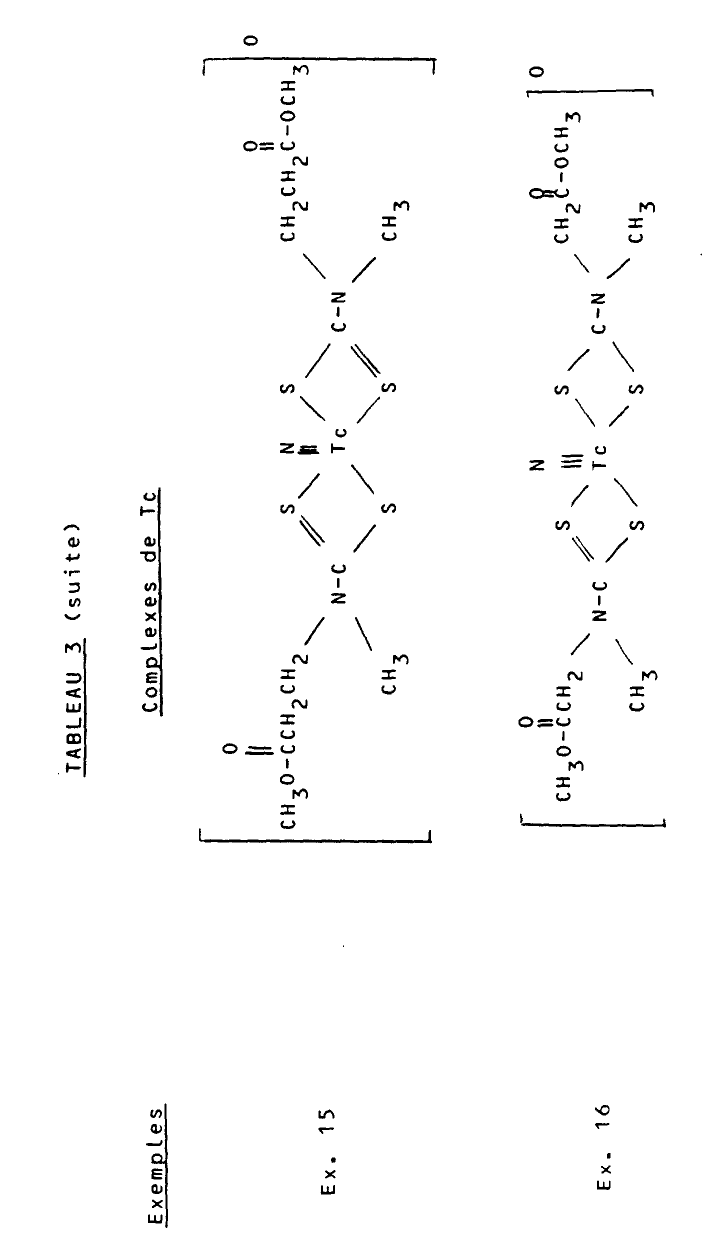

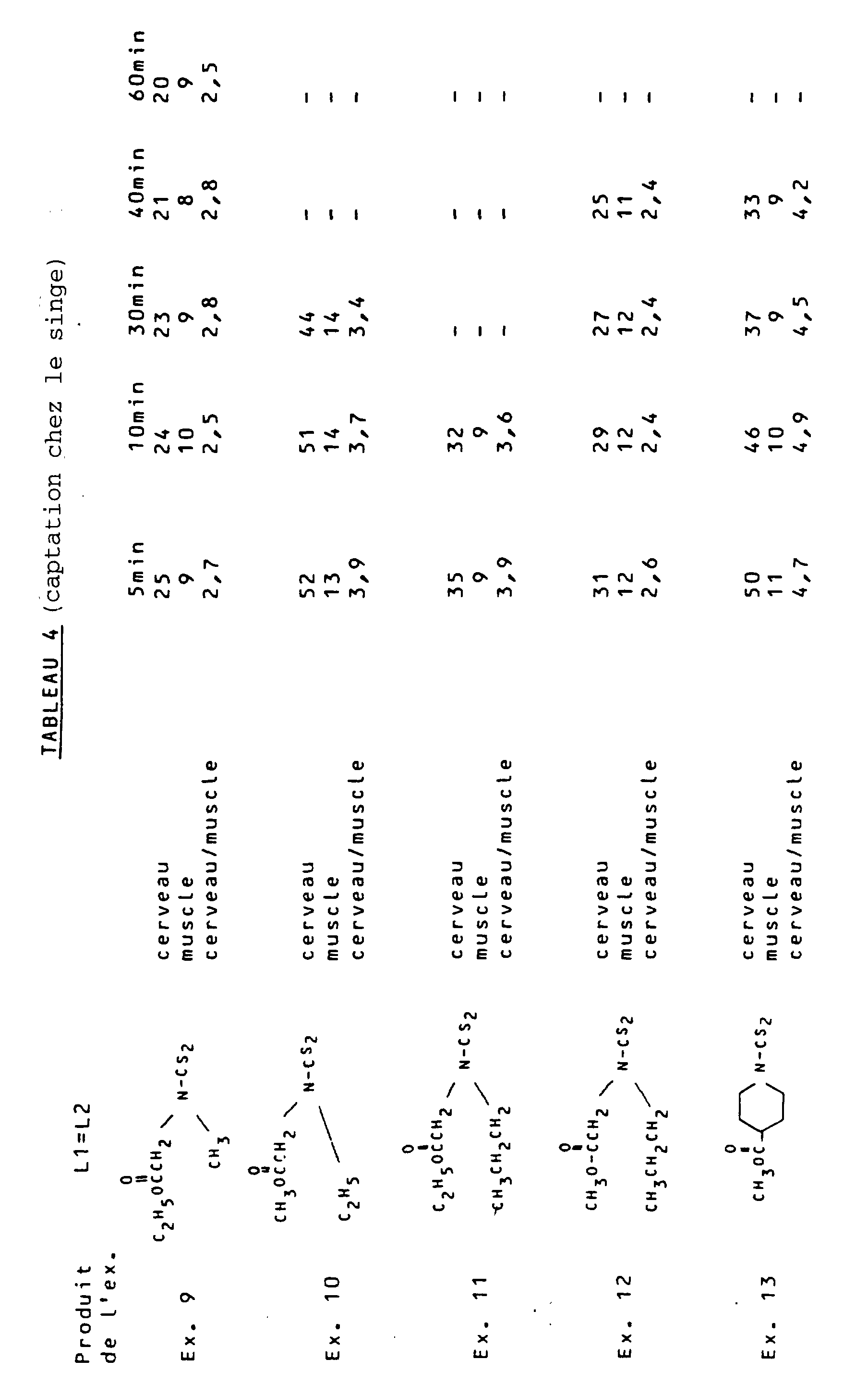

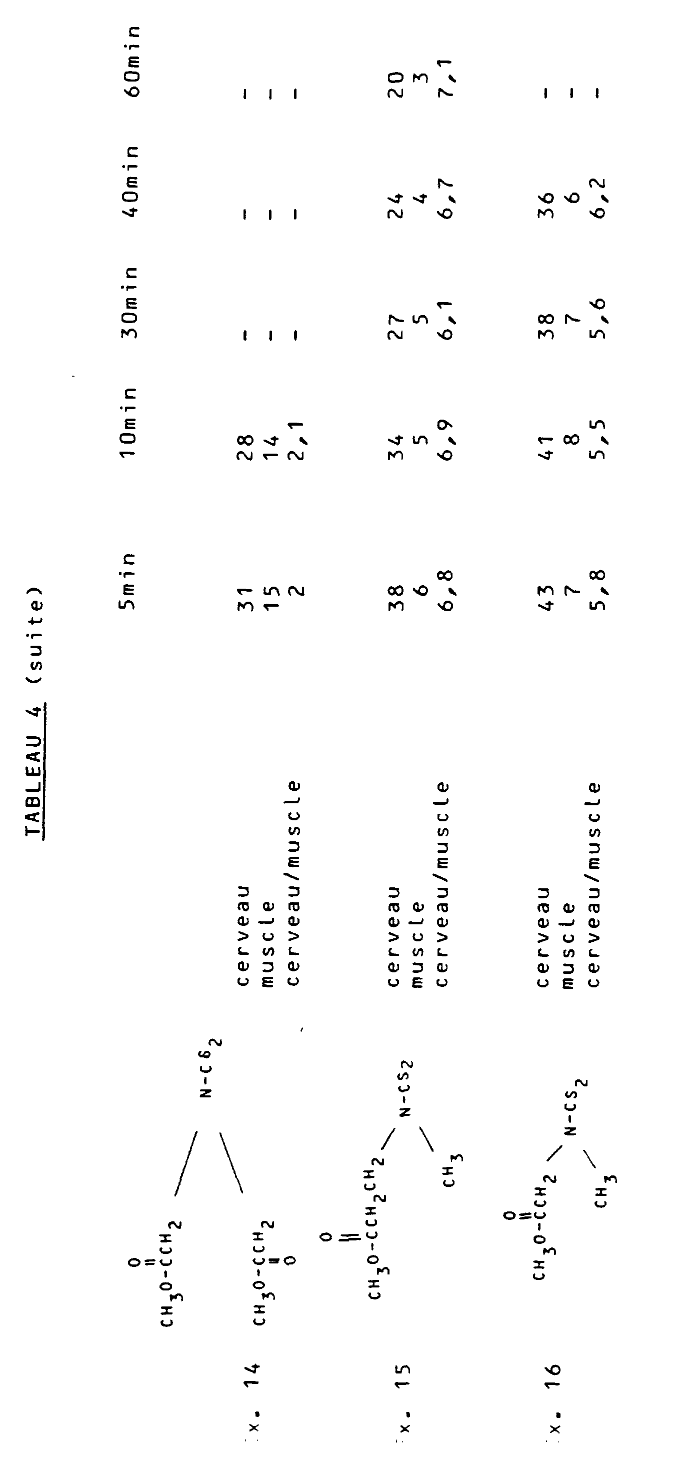

## Revendications (Revendications pour l'(les) Etat(s) contractant(s) suivant(s): AT, BE, CH, LI, DE, DK, FR, GB, IT, LU, NL, SE)

1. Produit radiopharmaceutique, caractérisé en ce qu'il comprend un complexe d'un métal de transition M répondant à la formule :
(M≡N) L¹ L² (I)
dans laquelle L¹ et L² qui peuvent être identiques ou différents, répondent à la formule : 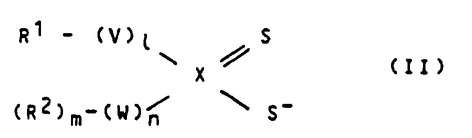 dans laquelle R¹ et R² qui peuvent être identiques ou différents, représentent un radical alkyle, linéaire ou ramifié de 1 à 10 atomes de carbone, non substitué ou substitué par au moins un groupement choisi parmi les groupements -O-R³, -OOCR³, -COOR³, -OCNR⁴R⁵ ou -NR⁴R⁵ dans lesquels R³ est un radical alkyle linéaire ou ramifié de 1 à 5 atomes de carbone, et R⁴ et R⁵ qui peuvent être identiques ou différents, sont des atomes d'hydrogène ou des radicaux alkyle, linéaires ou ramifiés, de 1 à 5 atomes de carbone, ou dans laquelle R¹ et R² forment ensemble un cycle hydrocarboné contenant éventuellement un ou plusieurs hétéroatomes, non substitué ou substitué par au moins un groupement choisi parmi les groupements -O-R³, -OOCR³, -COOR³, -OCNR⁴R⁵ ou -NR⁴R⁵ dans lesquels R³, R⁴ et R⁵ sont tels que définis ci-dessus,
- V et W qui peuvent être identiques ou différents, représentent O, S ou Se,
- X représente C, N-C, P ou As, et
- l, m et n, qui peuvent être identiques ou différents, sont égaux à 0 ou à 1, m et n étant égaux à 0 Lorsque X représente C, et m étant égal à 1 Lorsque X représente N-C, P ou As,
à condition que
1°) lorsque X représente -N-C-, R¹ ou R² soit un radical alkyle substitué par au moins un groupement -COOR³, ou R¹ et R² forment ensemble un cycle hydrocarboné substitué, contenant éventuellement un ou plusieurs hétéroatomes, et
2°) Lorsque X représente P avec V et W représentant O, R¹ ou R² soit un radical alkyle, substitué par au moins un groupement choisi parmi les groupements OR³, OOCR³, COOR³, OCNR⁴R⁵ ou NR⁴R⁵ avec R³, R⁴ et R⁵ tels que définis ci-dessus, ou R¹ et R² forment ensemble un cycle hydrocarboné substitué, contenant éventuellement un ou plusieurs hétéroatomes.

2. Produit radiopharmaceutique selon la revendication 1, caractérisé en ce que L¹ et/ou L² répondent à la formule :

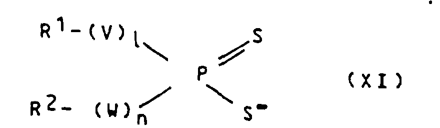

3. Produit radiopharmaceutique selon la revendication 2, caractérisé en ce que l et n sont égaux à 0.

4. Produit radiopharmaceutique selon la revendication 3, caractérisé en ce que R¹ et R² sont des radicaux méthyle ou éthyle.

5. Produit radiopharmaceutique selon la revendication 1, caractérisé en ce que
L¹ représente 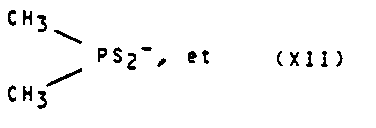
L² représente

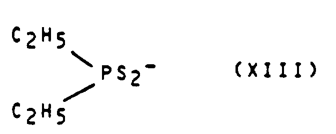

6. Produit radiopharmaceutique selon la revendication 1, caractérisé en ce que L¹ et/ou L² répondent à la formule : 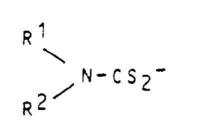 dans laquelle R¹ est un radical alkyle substitué par -COOR³ et R² est un radical alkyle non substitué ou substitué par -COOR³.

7. Produit radiopharmaceutique selon la revendication 1, caractérisé en ce que L¹ et/ou L² répondent à l'une des formules suivantes : 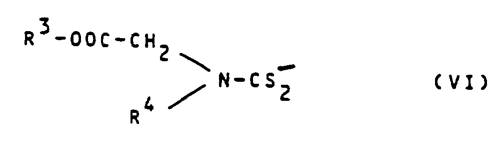 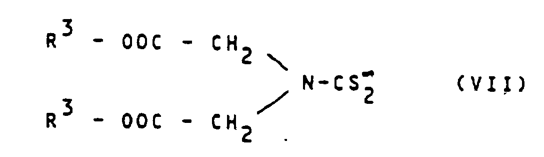 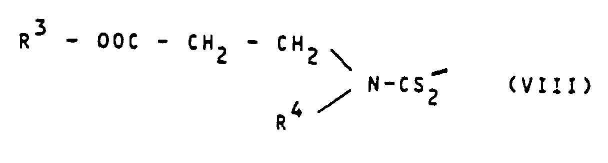 dans lesquelles R³ est le radical méthyle ou éthyle et R⁴ est le radical méthyle, éthyle ou propyle.

8. Produit radiopharmaceutique selon la revendication 1, caractérisé en ce que L¹ et/ou L² répondent à la formule : 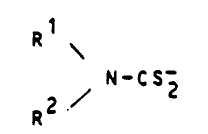 dans laquelle R¹ et R² forment ensemble un cycle hydrocarboné substitué par un groupement -COOR³ avec R³ représentant un radical alkyle linéaire ou ramifié de 1 à 5 atomes de carbone.

9. Produit radiopharmaceutique selon l'une quelconque des revendications 1 à 8, caractérisé en ce que L¹ et L² sont identiques.

10. Produit radiopharmaceutique selon l'une quelconque des revendications 1 à 9, caractérisé en ce que M représente un isotope du technétium ou du rhénium.

11. Produit radiopharmaceutique selon la revendication 10, caractérisé en ce que l'isotope du technétium est Tc99m.

12. Produit radiopharmaceutique selon la revendication 10, caractérisé en ce que l'isotope du rhénium est Re-186 ou Re-188.

13. Produit radiopharmaceutique, caractérisé en ce qu'il est choisi parmi le nitruro-bis(diméthyl dithiophosphinato)^{99m}Tc (V), le nitruro-bis (diéthyl dithiophosphinato)^{99m}Tc (V), le nitruro(diméthyldithiophosphinato, diéthyldithiophosphinato)^{99m}Tc (V), le nitruro bis (N-(N,N diméthyléthylamino), N-méthyl dithiocarbamato)^{99m}TC (V), le nitruro bis (N-(N,N diéthyléthylamino), N-méthyl dithiocarbamato)^{99m}Tc (V), le nitruro bis (N-méthylèneméthylcarboxylate, N-méthyl dithiocarbamato)^{99m}Tc (V), le nitruro bis (N-méthylène éthyl carboxylate, N-méthyt dithiocarbamato)^{99m}Tc (V), le nitruro bis (N-méthylène méthyl carboxylate, N-éthyl dithiocarbamato)^{99m}Tc (V), le nitruro bis (N-méthylène méthyl carboxylate, N-propyl dithiocarbamato)^{99m}Tc (V), le nitruro bis (N-méthylène éthyl carboxylate, N-n-propyl dithiocarbamato)^{99m}Tc (V), le nitruro bis (3-(méthyl carboxylate)pipéridino dithiocarbamato)^{99m}Tc (V), le nitruro bis [bis N-(méthylène méthyl carboxylate)]dithiocarbamato)^{99m}Tc (V), et le nitruro bis (N-éthylène-2-(méthyl carboxylate), N-méthyl dithiocarbamato)^{99m}Tc (V).

14. Procédé de préparation d'un produit radiopharmaceutique selon L'une quelconque des revendications 1 à 13, caractérisé en ce qu'il comprend les étapes successives suivantes :
1°) faire réagir en solution un composé oxygéné d'un métal de transition M avec :
a) un premier réactif choisi dans le groupe des phosphines et polyphosphines aliphatiques et aromatiques, substituées ou non substituées, et
b) un second réactif choisi parmi les azotures d'ammonium et de métaux pharmaceutiquement acceptables et les ligands azotés comportant un motif 〉N-N〈 dans lesquels les N sont reliés à des atomes d'hydrogène et/ou à des groupements organiques monovalents, ou dans lequel l'un des N est relié à l'atome de carbone d'un groupe organique bivalent par l'intermédiaire d'une double liaison et l'autre N est relié à des atomes d'hydrogène et/ou à des groupements organiques monovalents, et
2°) faire réagir le produit intermédiaire obtenu dans la première étape avec un composé répondant à la formule : 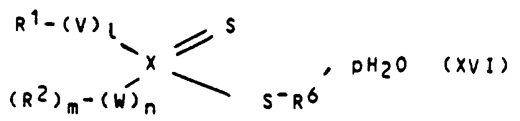 dans laquelle R¹, R², V, W, X, l, m et n ont la signification donnée dans la revendication 1,
R⁶ est un ion de métal alcalin, H⁺ ou NH₄⁺, et p est égal à 0 ou est un nombre entier allant de 1 à 5.

15. Procédé de préparation d'un produit radiopharmaceutique selon l'une quelconque des revendications 1 à 13, caractérisé en ce qu'il comprend les étapes suivantes :
1°) faire réagir en solution un composé oxygéné du métal de transition M avec
a) un ligand azoté constitué soit par un azoture d'ammonium ou de métal pharmaceutiquement acceptable, soit par un composé azoté comportant un motif 〉N-N dans lequel les N sont reliés à des atomes d'hydrogène et/ou à des groupements organiques monovalents, ou dans lequel l'un des N est relié à l'atome de carbone d'un groupement organique bivalent par l'intermédiaire d'une double liaison et l'autre N〈 est relié à des atomes d'hydrogène et/ou à des groupements organiques monovalents, et
b) un agent réducteur constitué, soit par du dithionite d'ammonium ou d'un métal pharmaceutiquement acceptable, soit par de l'étain (II) présent sous forme ionique dans la solution, et
2°) faire réagir le produit intermédiaire obtenu dans la première étape avec un composé répondant à la formule 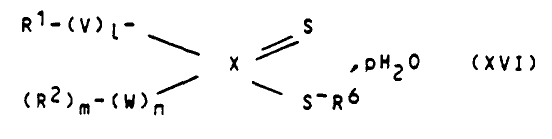 dans laquelle R¹, R², V, W, l, m, n et X ont la signification donnée dans la revendication 1,
R⁶ est un ion de métal alcalin, H⁺ ou NH₄⁺, et p est égal à 0 ou est un nombre entier allant de 1 à 5.

16. Procédé selon l'une quelconque des revendications 14 et 15, caractérisé en ce que le composé oxygéné du métal de transition est un pertechnétate de métal alcalin ou d'ammonium.

17. Procédé selon l'une quelconque des revendications 14 et 15, caractérisé en ce que le composé oxygéné du métal de transition est du perrhénate de métal alcalin ou d'ammonium.

18. Procédé selon la revendication 14, caractérisé en ce que le premier réactif est une phosphine choisie parmi la triphénylphosphine, la diéthylphénytphosphine, la triéthylphosphine, la triméthylphosphine, la tris(2-cyanoéthyl)phosphine et la triphénylphosphine trisulfonée.

19. Procédé selon l'une quelconque des revendications 14 et 15, caractérisé en ce que le ligand azoté est choisi parmi le S-méthyldithiocarbazate, le S-méthyl-N-méthyldithiocarbazate, l'alpha--N-méthyl-S-méthyl bêta-N-pyridylméthylène dithiocarbazate, le S-méthyl-bêta-N-(2-hydroxyphényl)méthylène dithiocarbazate et le alpha-N-méthyl-S-méthyl-bêta-N-(2-hydroxyphényl)méthylène dithiocarbazate.

20. Procédé selon l'une quelconque des revendications 14 et 15, caractérisé en ce que le ligand azoté répond à la formule : 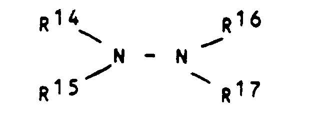
dans laquelle R¹⁴, R¹⁵ et R¹⁶ qui peuvent être identiques ou différents, représentent un atome d'hydrogène, un radical alkyle, un radical aryle, un radical alcoxy, un radical alkyle substitué par au moins un groupement choisi parmi les radicaux hydroxy, carboxy, amino, amido et mercapto ou un radical aryle substitué par au moins un groupement choisi parmi les atomes d'halogène et les radicaux alcoxy, hydroxy, amino, mercapto et amino substitué par au moins un radical alkyle,
R¹⁷ représente un radical répondant aux formules :
-SO₂R¹⁸ ou -COR¹⁹
dans lesquelles R¹⁸ est un groupe phényle non substitué ou substitué par au moins un substituant choisi parmi les atomes d'halogène et les radicaux alkyle, et
R¹⁹ est un atome d'hydrogène, -NH₂ ou un radical choisi parmi les radicaux alkyle, les radicaux alkyle substitués par au moins un groupement choisi parmi les groupements cyano, pyridyle et -CO-NH-NH₂, le radical phényle, le radical phényle substitué par au moins un substituant choisi parmi -OH, NH₂ et les radicaux alkyle et alcoxy.

21. Procédé selon la revendication 20, caractérisé en ce que R¹⁴, R¹⁵ et R¹⁶ sont des atomes d'hydrogène, R¹⁷ représente le radical de formule SO₂R¹⁸ avec R¹⁸ ayant la signification donnée dans la revendication 18, ou le radical de formule COH, CO-CH₂-CH₂-CO-NH-NH₂ ou 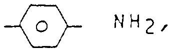 et en ce que l'on effectue la réaction à la température ambiante.

22. Procédé selon la evendication 21, caractérisé en ce que R¹⁷ représente SO₂R¹⁸ avec R¹⁸ étant le radical phényle, le radical p-méthylphényle, le radical trichloro-1,3,5 phényle ou le radical triméthyl-1,3,5 phényle.

23. Procédé selon l'une quelconque des revendications 14 et 16 à 18, caractérisé en ce que le second réactif est l'azoture de sodium.

24. Trousse pour la préparation d'un produit radiopharmaceutique, caractérisée en ce qu'elle comprend :
- un premier flacon contenant une phosphine ou un agent réducteur choisi parmi le dithionite d'ammonium, les dithionites de métaux pharmaceutiquement acceptables et l'étain (II) sous forme ionique,
- un second flacon contenant de l'azoture d'ammonium, de l'azoture d'un métal pharmaceutiquement acceptable, ou un ligand azoté, et
- un troisième flacon contenant un composé répondant à la formule : 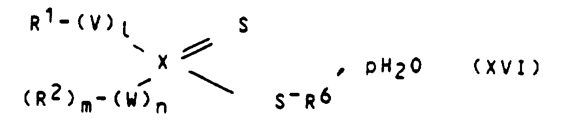 dans laquelle R¹, R², V, W, l, m, n et X ont la signification donnée dans la revendication 1, R⁶ est un ion de métal alcalin, H⁺ ou NH₄⁺, et p est égal à 0 ou est un nombre entier allant de 1 à 5.

25. Utilisation du complexe de métal de transition obtenu par le procédé selon l'une quelconque des revendications 14 à 23 pour la fabrication d'un produit radiopharmaceutique à tropisme cérébral.

26. Produit radiopharmaceutique selon l'une quelconque des revendications 1 à 13, caractérisé en ce qu'il présente un tropisme cérébral.

## Revendications (Revendications pour l'(les) Etat(s) contractant(s) suivant(s): ES, GR)

1. Procédé de préparation d'un produit radiopharmaceutique comprenant un complexe d'un métal de transition M répondant à la formule :
(M≡N) L¹ L² (I)
dans laquelle L¹ et L² qui peuvent être identiques ou différents, répondent à la formule : 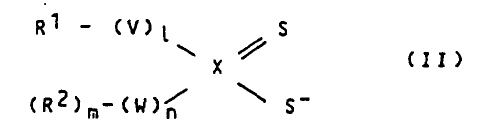 dans laquelle R¹ et R² qui peuvent être identiques ou différents, représentent un radical alkyle, linéaire ou ramifié de 1 à 10 atomes de carbone, non substitué ou substitué par au moins un groupement choisi parmi les groupements -O-R³, -OOCR³, -COOR³, -OCNR⁴R⁵ ou -NR⁴R⁵ dans lesquels R³ est un radical alkyle linéaire ou ramifié de 1 à 5 atomes de carbone, et R⁴ et R⁵ qui peuvent être identiques ou différents, sont des atomes d'hydrogène ou des radicaux alkyle, linéaires ou ramifiés, de 1 à 5 atomes de carbone, ou dans laquelle R¹ et R² forment ensemble un cycle hydrocarboné contenant éventuellement un ou plusieurs hétéroatomes, non substitué ou substitué par au moins un groupement choisi parmi les groupements -O-R³, -OOCR³, -COOR³, -OCNR⁴R⁵ ou -NR⁴R⁵ dans lesquels R³, R⁴ et R⁵ sont tels que définis ci-dessus,
- V et W qui peuvent être identiques ou différents, représentent O, S ou Se,
- X représente C, N-C, P ou As, et
- l, m et n, qui peuvent être identiques ou différents, sont égaux à 0 ou à 1, m et n étant égaux à 0 lorsque X représente C, et m étant égal à 1 lorsque X représente N-C, P ou As,
à condition que
1°) lorsque X représente -N-C-, R¹ ou R² soit un radical alkyle substitué par au moins un groupement -COOR³, ou R¹ et R² forment ensemble un cycle hydrocarboné substitué, contenant éventuellement un ou plusieurs hétéroatomes, et
2°) Lorsque X représente P avec V et W représentant O, R¹ ou R² soit un radical alkyle substitué par au moins un groupement choisi parmi les groupements OR³, OOCR³, COOR³, OCNR⁴R⁵ ou NR⁴R⁵ avec R³, R⁴ et R⁵ tels que définis ci-dessus, ou R¹ et R² forment ensemble un cycle hydrocarboné substitué, contenant éventuellement un ou plusieurs hétéroatomes, ledit procédé comprenant les étapes suivantes :
1°) faire réagir en solution un composé oxygéné du métal de transition M avec :
a) un premier réactif choisi dans le groupe des phosphines et polyphosphines aliphatiques et aromatiques, substituées ou non substituées, et
b) un second réactif choisi parmi les azotures d'ammonium et de métaux pharmaceutiquement acceptables et les ligands azotés comportant un motif 〉N-N〈 dans lesquels les N sont reliés à des atomes d'hydrogène et/ou à des groupements organiques monovalents, ou dans lequel l'un des N est relié à l'atome de carbone d'un groupe organique bivalent par l'intermédiaire d'une double liaison et l'autre N est relié à des atomes d'hydrogène et/ou à des groupements organiques monovalents, et
2°) faire réagir le produit intermédiaire obtenu dans la première étape avec un composé répondant à la formule : 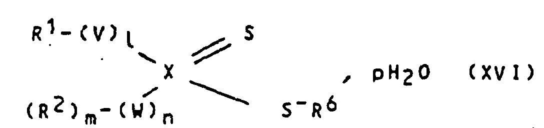 dans laquelle R¹, R², V, W, X, l, m et n ont la signification donnée ci-dessus,
R⁶ est un ion de métal alcalin, H⁺ ou NH₄⁺, et p est égal à 0 ou est un nombre entier allant de 1 à 5.

2. Procédé de préparation d'un produit radiopharmaceutique comprenant un complexe d'un métal de transition M répondant à la formule :
(M≡N) L¹ L² (I)
dans laquelle L¹ et L² qui peuvent être identiques ou différents, répondent à la formule : 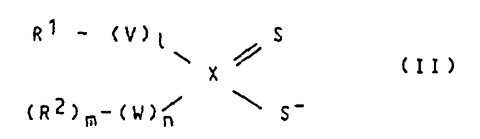 dans laquelle R¹ et R² qui peuvent être identiques ou différents, représentent un radical alkyle, linéaire ou ramifié de 1 à 10 atomes de carbone, non substitué ou substitué par au moins un groupement choisi parmi les groupements -O-R³, -OOCR³, -COOR³, -OCNR⁴R⁵ ou -NR⁴R⁵ dans lesquels R³ est un radical alkyle linéaire ou ramifié de 1 à 5 atomes de carbone, et R⁴ et R⁵ qui peuvent être identiques ou différents, sont des atomes d'hydrogène ou des radicaux alkyle, linéaires ou ramifiés, de 1 à 5 atomes de carbone, ou dans laquelle R¹ et R² forment ensemble un cycle hydrocarboné contenant éventuellement un ou plusieurs hétéroatomes, non substitué ou substitué par au moins un groupement choisi parmi les groupements -O-R³, -OOCR³, -COOR³, -OCNR⁴R⁵ ou -NR⁴R⁵ dans lesquels R³, R⁴ et R⁵ sont tels que définis ci-dessus,
- V et W qui peuvent être identiques ou différents, représentent O, S ou Se,
- X représente C, N-C, P ou As, et
- l, m et n, qui peuvent être identiques ou différents, sont égaux à 0 ou à 1, m et n étant égaux à 0 lorsque X représente C, et m étant égal à 1 lorsque X représente N-C, P ou As,
à condition que
1°) lorsque X représente -N-C-, R¹ ou R² soit un radical alkyle substitué par au moins un groupement -COOR³, ou R¹ et R² forment ensemble un cycle hydrocarboné substitué, contenant éventuellement un ou plusieurs hétéroatomes, et
2°) Lorsque X représente P avec V et W représentant O, R¹ ou R² soit un radical alkyle substitué par au moins un groupement choisi parmi les groupements OR³, OOCR³, COOR³, OCNR⁴R⁵ ou NR⁴R⁵ avec R³, R⁴ et R⁵ tels que définis ci-dessus, ou R¹ et R² forment ensemble un cycle hydrocarboné substitué, contenant éventuellement un ou plusieurs hétéroatomes, ledit procédé comprenant les étapes suivantes :
1°) faire réagir en solution un composé oxygéné du métal de transition M avec
a) un ligand azoté constitué soit par un azoture d'ammonium ou de métal pharmaceutiquement acceptable soit par un composé azoté comportant un motif 〉N N dans lequel les N sont reliés à des atomes d'hydrogène et/ou à des groupements organiques monovalents, ou dans lequel l'un des N est relié à l'atome de carbone d'un groupement organique bivalent par l'intermédiaire d'une double liaison et l'autre N〈 est relié à des atomes d'hydrogène et/ou à des groupements organiques monovalents, et
b) un agent réducteur constitué, soit par du dithionite d'ammonium ou d'un métal pharceutiquement acceptable, soit par de l'étain (II) présent sous forme ionique dans la solution, et
2°) faire réagir le produit intermédiaire obtenu dans la première étape avec un composé répondant à la formule 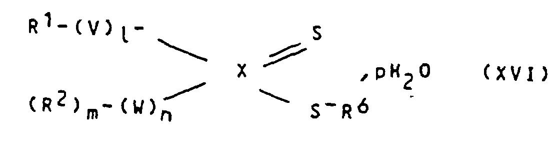 dans laquelle R¹, R², V, W, l, m, n et X ont la signification donnée ci-dessus,
R⁶ est un ion de métal alcalin, H⁺ ou NH₄⁺, et p est égal à 0 ou est un nombre entier allant de 1 à 5.

3. Procédé selon la revendication 1 ou 2, caractérisé en ce que L¹ et/ou L² répondent à la formule:

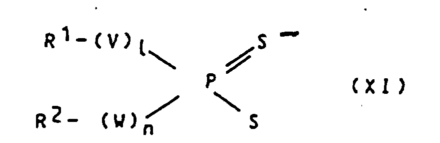

4. Procédé selon la revendication 3, caractérisé en ce que l et n sont égaux à 0.

5. Procédé selon la revendication 4, caractérisé en ce que R¹ et R² sont des radicaux méthyle ou éthyle.

6. Procédé selon la revendication 1 ou 2, caractérisé en ce que
L¹ représente 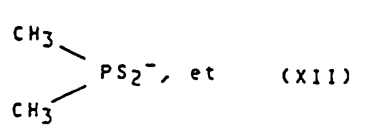
L² représente

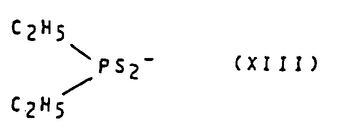

7. Procédé selon la revendication 1 ou 2, caractérisé en ce que L¹ et/ou L² répondent à la formule : 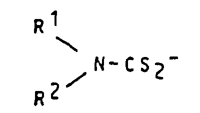 dans laquelle R¹ est un radical alkyle substitué par -COOR³ et R² est un radical alkyle non substitué ou substitué par -COOR³.

8. Procédé selon la revendication 1 ou 2, caractérisé en ce que L¹ et/ou L² répondent à l'une des formules suivantes : 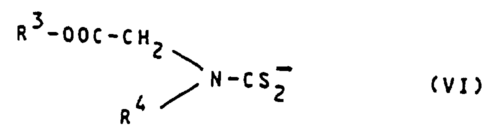 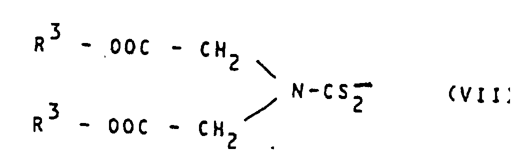 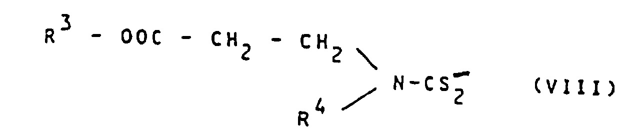 dans lesquelles R³ est le radical méthyle ou éthyle et R⁴ est le radical méthyle, éthyle ou propyle.

9. Procédé selon la revendication 1 ou 2, caractérisé en ce que L¹ et/ou L² répondent à la formule : 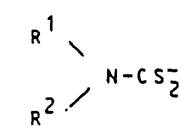 dans laquelle R¹ et R² forment ensemble un cycle hydrocarboné substitué par un groupement -COOR³ avec R³ représentant un radical alkyle linéaire ou ramifié de 1 à 5 atomes de carbone.

10. Procédé selon l'une quelconque des revendications 1 à 9, caractérisé en ce que L¹ et L² sont identiques.

11. Procédé selon l'une quelconque des revendications 1 à 10, caractérisé en ce que M représente un isotope du technétium ou du rhénium.

12. Procédé selon la revendication 11, caractérisé en ce que l'isotope du technétium est Tc99m.

13. Procédé selon la revendication 11, caractérisé en ce que l'isotope du rhénium est Re-186 ou Re-188.

14. Procédé selon la revendication 1 ou 2, dans lequel le produit radiopharmaceutique est choisi parmi le nitruro-bis(diméthyl dithiophosphinato)^{99m}Tc (V), le nitruro-bis (diéthyl dithiophosphinato)^{99m}Tc (V), le nitruro(diméthyldithiophosphinato, diéthyldithiophosphinato)^{99m}Tc (V), le nitruro bis (N-(N,N diméthyléthylamino), N-méthyl dithiocarbamato)^{99m}TC (V), le nitruro bis (N-(N,N diéthyléthylamino), N-méthyl dithiocarbamato)^{99m}Tc (V), le nitruro bis (N-méthylèneméthylcarboxylate, N-méthyl dithiocarbamato)^{99m}Tc (V), le nitruro bis (N-méthylène éthyl carboxylate, N-méthyl dithiocarbamato)^{99m}Tc (V), le nitruro bis (N-méthylène méthyl carboxylate, N-éthyl dithiocarbamato)^{99m}Tc (V), le nitruro bis (N-méthylène méthyl carboxylate, N-propyl dithiocarbamato)^{99m}Tc (V), le nitruro bis (N-méthylène éthyl carboxylate, N-n-propyl dithiocarbamato)^{99m}Tc (V), le nitruro bis. (3-(méthyl carboxylate)pipéridino dithiocarbamato)^{99m}Tc (V), le nitruro bis [bis N-(méthylène méthyl carboxylate)]dithiocarbamato)^{99m}Tc (V), et le nitruro bis (N-éthylène-2-(iéthyl carboxylate), N-méthyl dithiocarbamato)^{99m}Tc (V) .

15. Procédé selon l'une quelconque des revendications 1 à 14, caractérisé en ce que le composé oxygéné du métal de transition est un pertechnétate de métal alcalin ou d'ammonium.

16. Procédé selon l'une quelconque des revendications 1 à 14, caractérisé en ce que le composé oxygéné du métal de transition est du perrhénate de métal alcalin ou d'ammonium.

17. Procédé selon la revendication 1, caractérisé en ce que le premier réactif est une phosphine choisie parmi la triphénylphosphine, la diéthylphénylphosphine, la triéthylphosphine, la triméthylphosphine, la tris(2-cyanoéthyl)phosphine et la triphénylphosphine trisulfonée.

18. Procédé selon l'une quelconque des revendications 1 et 2, caractérisé en ce que le ligand azoté est choisi parmi le S-méthyldithiocarbazate, le S-méthyl-N-méthyldithiocarbazate, l'alpha--N-méthyl-S-méthyl bêta-N-pyridylméthylène dithiocarbazate, le S-méthyl-bêta-N-(2-hydroxyphényl)méthylène dithiocarbazate et le alpha-N-méthyl-s-méthyl-bêta-N-(2-hydroxyphényl)méthylène dithiocarbazate.

19. Procédé selon l'une quelconque des revendications 1 et 2, caractérisé en ce que le ligand azoté répond à la formule : 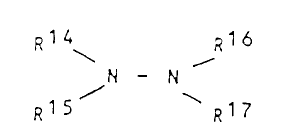
dans laquelle R¹⁴, R¹⁵ et R¹⁶ qui peuvent être identiques ou différents, représentent un atome d'hydrogène, un radical alkyle, un radical aryle, un radical alcoxy, un radical alkyle substitué par au moins un groupement choisi parmi les radicaux hydroxy, carboxy, amino, amido et mercapto ou un radical aryle substitué par au moins un groupement choisi parmi les atomes d'halogène et les radicaux alcoxy, hydroxy, amino, mercapto et amino substitué par au moins un radical alkyle,
R¹⁷ représente un radical répondant aux formules :
- SO₂R¹⁸ ou -COR¹⁹
dans lesquelles R¹⁸ est un groupe Phényle non substitué ou substitué par au moins un substituant choisi parmi les atomes d'halogène et les radicaux alkyle, et
R¹⁹ est un atome d'hydrogène, -NH₂ ou un radical choisi parmi les radicaux alkyle, les radicaux alkyle substitués par au moins un groupement choisi parmi les groupements cyano, pyridyle et -CO-NH-NH₂, le radical phényle, le radical phényle substitué par au moins un substituant choisi parmi -OH, NH₂ et les radicaux alkyle et alcoxy.

20. Procédé selon la revendication 19, caractérisé en ce que R¹⁴, R¹⁵ et R¹⁶ sont des atomes d'hydrogène, R¹⁷ représente le radical de formule SO₂R¹⁸ avec R¹⁸ ayant la signification donnée dans La revendication 18, ou le radical de formule COH, CO-CH₂-CH₂-CO-NH-NH₂ ou 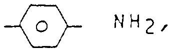 et en ce que l'on effectue la réaction à la température ambiante.

21. Procédé selon la revendication 20, caractérisé en ce que R¹⁷ représente le radical de formule SO₂R¹⁸ avec R¹⁸ étant le radical phényle, le radical p-méthylphényle, le radical trichloro-1,3,5 phényle ou le radical triméthyl-1,3,5 phényle.

22. Procédé selon l'une quelconque dès revendications 1 et 15 à 17, caractérisé en ce que le second réactif est l'azoture de sodium.

23. Trousse pour la préparation d'un produit radiopharmaceutique, caractérisée en ce qu'elle comprend :
- un premier flacon contenant une phosphine ou un agent réducteur choisi parmi le dithionite d'ammonium, les dithionites de métaux pharmaceutiquement acceptables et l'étain (II) sous forme ionique,
- un second flacon contenant de l'azoture d'ammonium, de l'azoture d'un métal pharmaceutiquement acceptable, ou un ligand azoté, et
- un troisième flacon contenant un composé répondant à la formule : 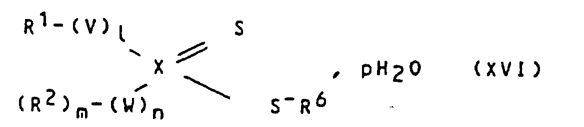 dans laquelle R¹, R², V, W, l, m, n et X ont la signification donnée dans la revendication 1, R⁶ est un ion de métal alcalin, H⁺ ou NH₄⁺, et p est égal à 0 ou est un nombre entier allant de 1 à 5.

24. Utilisation d'un complexe de métal de transition obtenu par le procédé selon l'une quelconque des revendications 14 à 22 pour la fabrication d'un produit radiopharmaceutique à tropisme cérébral.

25. Procédé selon l'une quelconque des revendications 1 à 22, dans lequel le produit radiopharmaceutique présente un tropisme cérébral.

## Patentansprüche (Patentansprüche für folgende(n) Vertragsstaat(en): AT, BE, CH, LI, DE, DK, FR, GB, IT, LU, NL, SE)

1. Radiopharmazeutisches Produkt, dadurch gekennzeichnet, dass es einen Komplex eines Übergangsmetalls M entsprechend der Formel:
(M≡N) L¹ L² (I)
umfasst, in der L¹ und L², die gleich oder verschieden sein können, der Formel: 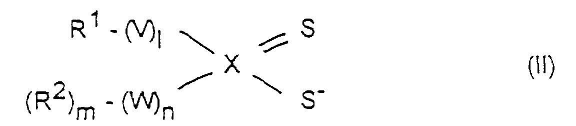 entsprechen, worin R¹ und R², die gleich oder verschieden sein können, einen linearen oder verzweigten Alkylrest von 1 bis 10 Kohlenstoffatomen darstellen, der nicht substituiert oder durch mindestens eine unter den Gruppen -OR³, -OOCR³, -COOR³, -OCNR⁴R⁵ oder -NR⁴R⁵, in denen R³ ein linearer oder verzweigter Alkylrest von 1 bis 5 Kohlenstoffatomen ist und R⁴ und R⁵, die gleich oder verschieden sein können, Waserstoffatome oder lineare oder verzweigte Alkylreste von 1 bis 5 Kohlenstoffatomen sind, gewählte Gruppe substituiert ist, oder worin R¹ und R² zusammen einen gegebenenfalls ein oder mehrere Heteroatome enthaltenden Kohlenwasserstoffring bilden, der nicht substituiert oder durch mindestens eine unter den Gruppen -OR³, -OOCR³, -COOR³, -OCNR⁴R⁵ oder -NR⁴R⁵, worin R³, R⁴ und R⁵ wie oben definiert sind, gewählte Gruppe substituiert ist,
- V und W, die gleich oder verschieden sein können, O, S oder Se darstellen,
- X C, N-C, P oder As darstellt und
- l, m und n, die gleich oder verschieden sein können, gleich 0 oder 1 sind, wobei m und n gleich 0 sind, wenn X C darstellt, und m gleich 1 ist, wenn X N-C, P oder As darstellt,
unter der Bedingung, dass,
1°) wenn X -N-C- darstellt, R¹ oder R² ein durch mindestens eine -COOR³-Gruppe substituierter Alkylrest ist oder R¹ und R² zusammen einen substituierten Kohlenwasserstoffring bilden, der gegebenenfalls ein oder mehrere Heteroatome enthält, und,
2°) wenn X P darstellt, mit V und W, die O darstellen, R¹ oder R² ein durch eine unter den Gruppen -OR³, -OOCR³, -COOR³, -OCNR⁴R⁵ oder -NR⁴R⁵ gewählte Gruppe substituierter Alkylrest ist, wobei R³, R⁴ und R⁵ wie oben definiert sind, oder R¹ und R² zusammen einen substituierten Kohlenwasserstoffring bilden, der gegebenenfalls ein oder mehrere Heteroatome enthält.

2. Radiopharmazeutisches Produkt nach Anspruch 1, dadurch gekennzeichnet, dass L¹ und/oder L² der Formel: 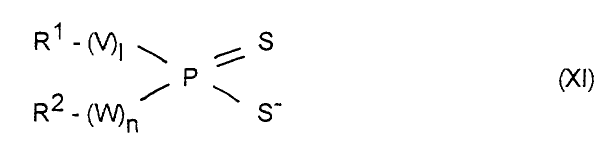 entsprechen.

3. Radiopharmazeutisches Produkt nach Anspruch 2, dadurch gekennzeichnet, dass I und n gleich 0 sind.

4. Radiopharmazeutisches Produkt nach Anspruch 3, dadurch gekennzeichnet, dass R¹ und R² Methyl- oder Ethylreste sind.

5. Radiopharmazeutisches Produkt nach Anspruch 1, dadurch gekennzeichnet, dass L¹ 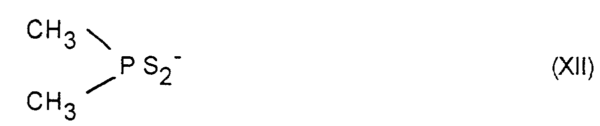 darstellt und L² 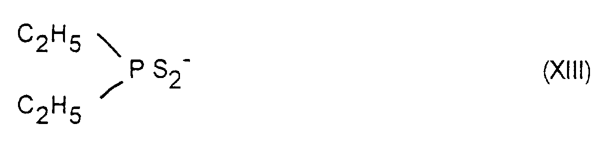 darstellt.

6. Radiopharmazeutisches Produkt nach Anspruch 1, dadurch gekennzeichnet, dass L¹ und/oder L² der Formel: 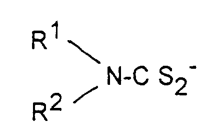 entsprechen, worin R¹ ein durch -COOR³ substituierter Alkylrest und R² ein nicht substituierter oder durch -COOR³ substituierter Alkylrest ist.

7. Radiopharmazeutisches Produkt nach Anspruch 1, dadurch gekennzeichnet, dass L¹ und/oder L² einer der folgenden Formeln entsprechen: 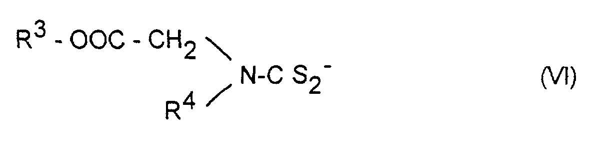 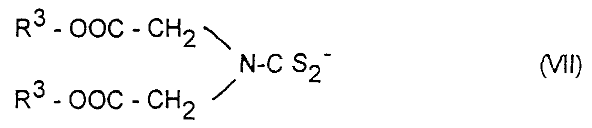 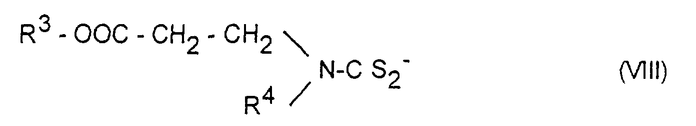 in denen R³ der Methyl- oder Ethylrest ist und R⁴ der Methyl-, Ethyl- oder Propylrest ist.

8. Radiopharmazeutisches Produkt nach Anspruch 1, dadurch gekennzeichnet, dass L¹ und/oder L² der Formel: 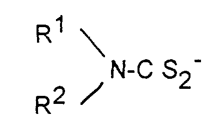 entsprechen, worin R¹ und R² zusammen einen Kohlenwasserstoffring bilden, der durch eine -COOR³-Gruppe substituiert ist, wobei R³ einen linearen oder verzweigten Alkylrest von 1 bis 5 Kohlenstoffatomen darstellt.

9. Radiopharmazeutisches Produkt nach einem der Ansprüche 1 bis 8, dadurch gekennzeichnet, dass L¹ und L² gleich sind.

10. Radiopharmazeutisches Produkt nach einem der Ansprüche 1 bis 9, dadurch gekennzeichnet, dass M ein Isotop des Technetiums oder des Rheniums darstellt.

11. Radiopharmazeutisches Produkt nach Anspruch 10, dadurch gekennzeichnet, dass das Technetiumisotop Tc-99m ist.

12. Radiopharmazeutisches Produkt nach Anspruch 10, dadurch gekennzeichnet, dass das Rheniumisotop Re-186 oder Re-188 ist.

13. Radiopharmazeutisches Produkt, dadurch gekennzeichnet, dass es unter Nitrido-bis(dimethyldithiophosphinato)^{99m}Tc(V), Nitrido-bis(diethyldithiophosphinato)^{99m}Tc(V), Nitrido-dimethyldithiophosphinato-diethyldithiophosphinato^{99m}Tc(V), Nitrido-bis(N-(N,N-dimethylethylamino)-N-methyl-dithiocarbamato)^{99m}Tc(V), Nitrido-bis(N-(N,N-diethylethylamino)-N-methyl-dithiocarbamato)^{99m}Tc(V), Nitrido-bis(N-methoxycarbonylmethyl-N-methyl-dithiocarbamato)^{99m}Tc(V), Nitrido-bis(N-ethoxycarbonylmethyl-N-methyl-dithiocarbamato)^{99m}Tc(V), Nitrido-bis(N-methoxycarbonylmethyl-N-ethyl-dithiocarbamato)-^{99m}Tc(V), Nitrido-bis(N-methoxycarbonylmethyl-N-propyl-dithiocarbamato)-^{99m}Tc(V), Nitrido-bis(N-ethoxycarbonylmethyl-N-n-propyl-dithiocarbamato)-^{99m}Tc(V), Nitrido-bis[(4-methyoxycarbonyl-piperidin-1-yl)-dithiocarbonato]-^{99m}Tc(V), Nitrido-bis(bis(N-methoxycarbonylmethyl]-dithiocarbamato)^{99m}Tc(V) und Nitrido-bis[N-(2-methoxycarbonyl-ethyl)-N-methyl-dithiocarbamato]^{99m}Tc(V) gewählt ist.

14. Verfahren zur Herstellung eines radiopharmazeutischen Produkts nach einem der Ansprüche 1 bis 13, dadurch gekennzeichnet, dass es nacheinander die folgenden Schritte umfasst:
1°) in Lösung eine sauerstoffhaltige Verbindung eines Übergangsmetalls M reagieren zu lassen mit
a) einem ersten Reagenz, gewählt in der Gruppe der substituierten oder nicht substituierten aliphatischen und aromatischen Phosphine und Polyphosphine, und mit
b) einem zweiten Reagenz, gewählt unter den Aziden des Ammoniums und pharmazeutisch annehmbarer Metalle und den stickstoffhaltigen, ein Strukturmotiv 〉N-N〈 enthaltenden Liganden, in welchen die N an Wasserstoffatome und/oder an einwertige organische Gruppen gebunden sind oder in welchen das eine N über eine Doppelbindung an das Kohlenstoffatom einer zweiwertigen organischen Gruppe gebunden ist und das andere N an Wasserstoffatome und/oder an einwertige organische Gruppen gebunden ist, und
2°) das in dem ersten Schritt erhaltene Zwischenprodukt mit einer Verbindung reagieren zu lassen, die der Formel: 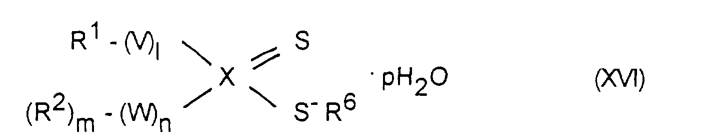 entspricht, worin R¹, R², V, W, X, l, m und n die in Anspruch 1 gegebene Bedeutung haben, R⁶ ein Alkalimetallion, H⁺ oder NH₄⁺ ist und p gleich 0 oder eine von 1 bis 5 gehende ganze Zahl ist.

15. Verfahren zur Herstellung eines radiopharmazeutischen Produkts nach einem der Ansprüche 1 bis 13, dadurch gekennzeichnet, dass es die folgenden Schritte umfasst:
1°) in Lösung eine sauerstoffhaltige Verbindung des Übergangsmetalls M reagieren zu lassen mit
a) einem stickstoffhaltigen Liganden, bestehend entweder aus einem Azid des Ammoniums oder eines pharmazeutisch annehmbaren Metalls oder aber aus einer stickstoffhaltigen, ein Strukturmotiv 〉N-N〈 enthaltenden Verbindung, in welcher die N an Wasserstoffatome und/oder an einwertige organische Gruppen gebunden sind oder in welcher das eine N über eine Doppelbindung an das Kohlenstoffatom einer zweiwertigen organischen Gruppe gebunden ist und das andere N〈 an Wasserstoffatome und/oder an einwertige organische Gruppen gebunden ist, und mit
b) einem Reduktionsmittel, das entweder aus Ammoniumdithionit oder dem Dithionit eines pharmazeutisch annehmbaren Metalls oder aus in der Lösung in lonenform vorhandenem Zinn(II) besteht, und
2°) das in dem ersten Schritt erhaltene Zwischenprodukt mit einer Verbindung reagieren zu lassen, die der Formel: 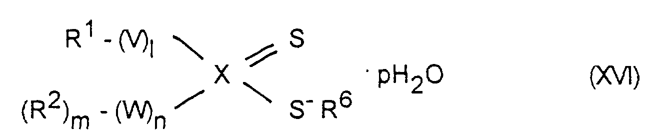 entspricht, worin R¹, R², V, W, l, m, n und X die in Anspruch 1 gegebene Bedeutung haben, R⁶ ein Alkalimetallion, H⁺ oder NH₄⁺ ist und p gleich 0 oder eine von 1 bis 5 gehende ganze Zahl ist.

16. Verfahren nach einem der Ansprüche 14 und 15, dadurch gekennzeichnet, dass die sauerstoffhaltige Verbindung des Übergangsmetalls ein Pertechnat eines Alkalimetalls oder des Ammoniums ist.

17. Verfahren nach einem der Ansprüche 14 und 15, dadurch gekennzeichnet, dass die sauerstoffhaltige Verbindung des Übergangsmetalls ein Perrhenat eines Alkalimetalls oder des Ammoniums ist.

18. Verfahren nach Anspruch 14, dadurch gekennzeichnet, dass das erste Reagenz ein Phosphin ist, gewählt unter Triphenylphosphin, Diethylphenylphosphin, Triethylphosphin, Trimethylphosphin, Tris(2-cyanoethyl)phosphin und dem trisulfonierten Triphenylphosphin.

19. Verfahren nach einem der Ansprüche 14 und 15, dadurch gekennzeichnet, dass der stickstoffhaltige Ligand gewählt ist unter S-Methyl-dithiocarbazat, S-Methyl-(2-methyldithiocarbazat), S-Methyl-(1-methyl-2-pyridylmethylen-dithiocarbazat), S-Methyl-[3-(2'-hydroxyphenyl)methylen-dithiocarbazat] und S-Methyl-[2-methyl-3-(2'-hydroxyphenyl)methylen-dithiocarbazat].

20. Verfahren nach einem der Ansprüche 14 und 15, dadurch gekennzeichnet, dass der stickstoffhaltige Ligand der Formel: 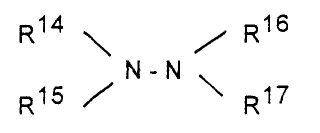 entspricht,
worin R¹⁴, R¹⁵ und R¹⁶, die gleich oder verschieden sein können, ein Wasserstoffatom, einen Alkylrest, einen Arylrest, einen Alkoxyrest, einen Alkylrest, welcher durch mindestens eine unter den Hydroxy-, Carboxy-, Amino-, Amido- und Mercaptoresten gewählte Gruppe substituiert ist, oder einen Arylrest, welcher durch mindestens eine unter den Halogenatomen und den Alkoxy-, Hydroxy-, Amino- und Mercaptoresten und den durch mindestens einen Alkylrest substituierten Aminoresten gewählte Gruppe substituiert ist, darstellen,
R¹⁷ einen Rest darstellt, der den Formeln:
-SO₂R¹⁸ oder -COR¹⁹
entspricht, worin R¹⁸ eine nicht substituierte oder durch mindestens einen unter den Halogenatomen und den Alkylresten gewählten Substituenten substituierte Phenylgruppe ist und
R¹⁹ ein Wasserstoffatom, -NH₂ oder ein Rest ist, der unter den Alkylresten, den durch mindestens eine unter den Cyano-, Pyridyl- und -CO-NH-NH₂-Gruppen gewählte Gruppe substituierten Alkylresten, dem Phenylrest, dem durch mindestens einen unter -OH, NH₂ und den Alkyl- und Alkoxyresten gewählten Substituenten substituierten Phenylrest gewählt ist.

21. Verfahren nach Anspruch 20, dadurch gekennzeichnet, dass R¹⁴, R¹⁵ und R¹⁶ Wasserstoffatome sind, R¹⁷ den Rest der Formel SO₂R¹⁸, wobei R¹⁸ die in Anspruch 18 gegebene Bedeutung hat, oder den Rest der Formel COH, CO-CH₂-CH₂-CO-NH-NH₂ oder 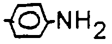 darstellt und dass man die Reaktion bei Umgebungstemperatur ausführt.

22. Verfahren nach Anspruch 21, dadurch gekennzeichnet, dass R¹⁷ SO₂R¹⁸ darstellt, wobei R¹⁸ der Phenylrest, der p-Methylphenylrest, der 1,3,5-Trichloro-phenylrest oder der 1,3,5-Trimethyl-phenylrest ist.

23. Verfahren nach einem der Ansprüche 14 und 16 bis 18, dadurch gekennzeichnet, dass das zweite Reagenz Natriumazid ist.

24. Garnitur für die Herstellung eines radiopharmazeutischen Produkts, dadurch gekennzeichnet, dass sie umfasst:
- eine erste Flasche, enthaltend ein Phosphin oder ein Reduktionsmittel, gewählt unter Ammoniumdithionit, den Dithioniten pharmazeutisch annehmbarer Metalle und Zinn(II) in lonenform,
- eine zweite Flasche, enthaltend Ammoniumazid, Azid eines pharmazeutisch annehmbaren Metalls oder einen stickstoffhaltigen Liganden, und
- eine dritte Flasche, enthaltend eine Verbindung entsprechend der Formel: 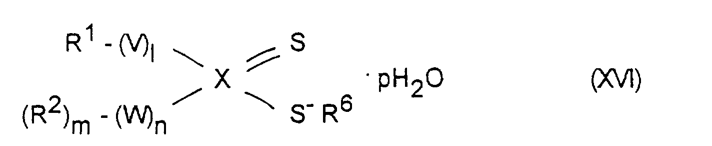 worin R¹, R², V, W, I, m, n und X die in Anspruch 1 gegebene Bedeutung haben, R⁶ ein Alkalimetallion, H⁺ oder NH₄⁺ ist und p gleich 0 oder eine von 1 bis 5 gehende ganze Zahl ist.

25. Verwendung des durch das Verfahren nach einem der Ansprüche 14 bis 23 erhaltenen Übergangsmetallkomplexes zur Herstellung eines radiopharmazeutischen Produkts mit zerebralem Affinität.

26. Radiopharmazeutisches Produkt nach einem der Ansprüche 1 bis 13, dadurch gekennzeichnet, dass es eine zerebrale Affinität aufweist.

## Patentansprüche (Patentansprüche für folgende(n) Vertragsstaat(en): ES, GR)

1. Verfahren zur Herstellung eines radiopharmazeutisches Produkts, das einen Komplex eines Übergangsmetalls M entsprechend der Formel:
(M≡N) L¹ L² (I)
enthält, in der L¹ und L², die gleich oder verschieden sein können, der Formel: 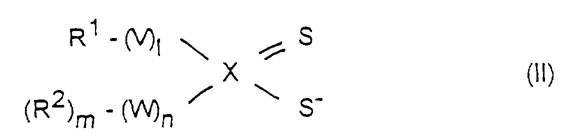 entsprechen, worin R¹ und R², die gleich oder verschieden sein können, einen linearen oder verzweigten Alkylrest von 1 bis 10 Kohienstoffatomen darstellen, der nicht substituiert oder durch mindestens eine unter den Gruppen -OR³, -OOCR³, -COOR³, -OCNR⁴R⁵ oder -NR⁴R⁵, in denen R³ ein linearer oder verzweigter Alkylrest von 1 bis 5 Kohlenstoffatomen ist und R⁴ und R⁵, die gleich oder verschieden sein können, Waserstoffatome oder lineare oder verzweigte Alkylreste von 1 bis 5 Kohlenstoffatomen sind, gewählte Gruppe substituiert ist, oder worin R¹ und R² zusammen einen gegebenenfalls ein oder mehrere Heteroatome enthaltenden Kohlenwasserstoffring bilden, der nicht substituiert oder durch mindestens eine unter den Gruppen -OR³, -OOCR³, -COOR³, -OCNR⁴R⁵ oder -NR⁴R⁵, worin R³, R⁴ und R⁵ wie oben definiert sind, gewählte Gruppe substituiert ist,
- V und W, die gleich oder verschieden sein können, O, S oder Se darstellen,
- X C, N-C, P oder As darstellt und
- l, m und n, die gleich oder verschieden sein können, gleich 0 oder 1 sind, wobei m und n gleich 0 sind, wenn X C darstellt, und m gleich 1 ist, wenn X N-C, P oder As darstellt,
unter der Bedingung, dass,
1°) wenn X -N-C- darstellt, R¹ oder R² ein durch mindestens eine -COOR³-Gruppe substituierter Alkylrest ist oder R¹ und R² zusammen einen substituierten Kohlenwasserstoffring bilden, der gegebenenfalls ein oder mehrere Heteroatome enthält, und,
2°) wenn X P darstellt, mit V und W, die O darstellen, R¹ oder R² ein durch mindestens eine unter den Gruppen -OR³, -OOCR³, -COOR³, -OCNR⁴R⁵ oder -NR⁴R⁵ gewählte Gruppe substituierter Alkylrest ist, wobei R³, R⁴ und R⁵ wie oben definiert sind, oder R¹ und R² zusammen einen substituierten Kohlenwasserstoffring bilden, der gegebenenfalls ein oder mehrere Heteroatome enthält, wobei das genannte Verfahren die folgenden Schritte umfasst:
1°). in Lösung eine sauerstoffhaltige Verbindung eines Übergangsmetalls M reagieren zu lassen mit
a) einem ersten Reagenz, gewählt in der Gruppe der substituierten oder nicht substituierten aliphatischen und aromatischen Phosphine und Polyphosphine, und mit
b) einem zweiten Reagenz, gewählt unter den Aziden des Ammoniums und pharmazeutisch annehmbarer Metalle und den stckstoffhaltigen, ein Strukturmotiv 〉N-N〈 enthaltenden Liganden, in welchen die N an Wasserstoffatome und/oder an einwertige organische Gruppen gebunden sind oder in welchen das eine N über eine Doppelbindung an das Kohlenstoffatom einer zweiwertigen organischen Gruppe gebunden ist und das andere N an Wasserstoffatome und/oder an einwertige organische Gruppen gebunden ist, und
2°) das in dem ersten Schritt erhaltene Zwischenprodukt mit einer Verbindung reagieren zu lassen, die der Formel: 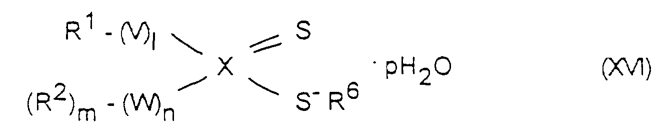 entspricht, worin R¹, R², V, W, X, I, m und n die oben gegebene Bedeutung haben, R⁶ ein Alkalimetallion, H⁺ oder NH₄⁺ ist und p gleich 0 oder eine von 1 bis 5 gehende ganze Zahl ist.

2. Verfahren zur Herstellung eines radiopharmazeutisches Produkts, das einen Komplex eines Übergangsmetalls M entsprechend der Formel:
(M ≡ N) L¹ L² (I)
enthält, in der L¹ und L², die gleich oder verschieden sein können, der Formel: 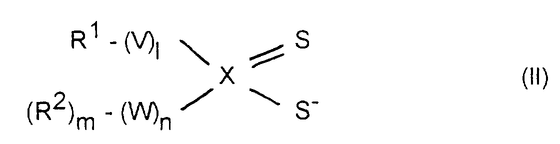 entsprechen, worin R¹ und R², die gleich oder verschieden sein können, einen linearen oder verzweigten Alkylrest von 1 bis 10 Kohlenstoffatomen darstellen, der nicht substituiert oder durch mindestens eine unter den Gruppen -OR³, -OOCR³, -COOR³, -OCNR⁴R⁵ oder -NR⁴R⁵, in denen R³ ein linearer oder verzweigter Alkylrest von 1 bis 5 Kohlenstoffatomen ist und R⁴ und R⁵, die gleich oder verschieden sein können, Waserstoffatome oder lineare oder verzweigte Alkylreste von 1 bis 5 Kohlenstoffatomen sind, gewählte Gruppe substituiert ist, oder worin R¹ und R² zusammen einen gegebenenfalls ein oder mehrere Heteroatome enthaltenden Kohlenwasserstoffring bilden, der nicht substituiert oder durch mindestens eine unter den Gruppen -OR³, -OOCR³, -COOR³, -OCNR⁴R⁵ oder -NR⁴R⁵, worin R³, R⁴ und R⁵ wie oben definiert sind, gewählte Gruppe substituiert ist,
- V und W, die gleich oder verschieden sein können, O, S oder Se darstellen,
- X C, N-C, P oder As darstellt und
- l, m und n, die gleich oder verschieden sein können, gleich 0 oder 1 sind, wobei m und n gleich 0 sind, wenn X C darstellt, und m gleich 1 ist, wenn X N-C, P oder As darstellt,
unter der Bedingung, dass,
1°) wenn X -N-C- darstellt, R¹ oder R² ein durch mindestens eine -COOR³-Gruppe substituierter Alkylrest ist oder R¹ und R² zusammen einen substituierten Kohlenwasserstoffring bilden, der gegebenenfalls ein oder mehrere Heteroatome enthält, und,
2°) wenn X P darstellt, mit V und W, die O darstellen, R¹ oder R² ein durch mindestens eine unter den Gruppen -OR³, -OOCR³, -COOR³, -OCNR⁴R⁵ oder -NR⁴R⁵ gewählte Gruppe substituierter Alkylrest ist, wobei R³, R⁴ und R⁵ wie oben definiert sind, oder R¹ und R² zusammen einen substituierten Kohlenwasserstoffring bilden, der gegebenenfalls ein oder mehrere Heteroatome enthält, wobei das genannte Verfahren die folgenden Schritte umfasst:
1°) in Lösung eine sauerstoffhaltige Verbindung des Übergangsmetalls M reagieren zu lassen mit
a) einem stickstoffhaltigen Liganden, bestehend entweder aus einem Azid des Ammoniums oder eines pharmazeutisch annehmbaren Metalls oder aber aus einer stickstoffhaltigen, ein Strukturmotiv 〉N-N〈 enthaltenden Verbindung, in welcher die N an Wasserstoffatome und/oder an einwertige organische Gruppen gebunden sind oder in welcher das eine N über eine Doppelbindung an das Kohlenstoffatom einer zweiwertigen organischen Gruppe gebunden ist und das andere N〈 an Wasserstoffatome und/oder an einwertige organische Gruppen gebunden ist, und mit
b) einem Reduktionsmittel, das entweder aus Ammoniumdithionit oder dem Dithionit eines pharmazeutisch annehmbaren Metalls oder aus in der Lösung in lonenform vorhandenem Zinn(II) besteht, und
2°) das in dem ersten Schritt erhaltene Zwischenprodukt mit einer Verbindung reagieren zu lassen, die der Formel: 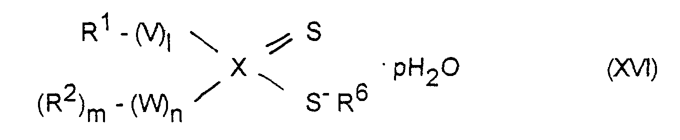 entspricht, worin R¹, R², V, W, X, l, m und n die oben gegebene Bedeutung haben, R⁶ ein Alkalimetallion, H⁺ oder NH₄⁺ ist und p gleich 0 oder eine von 1 bis 5 gehende ganze Zahl ist.

3. Verfahren nach Anspruch 1 oder 2, dadurch gekennzeichnet, dass L¹ und/oder L² der Formel: 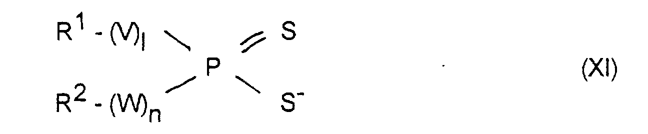 entsprechen.

4. Verfahren nach Anspruch 3, dadurch gekennzeichnet, dass I und n gleich 0 sind.

5. Verfahren nach Anspruch 4, dadurch gekennzeichnet, dass R¹ und R² Methyl- oder Ethylreste sind.

6. Verfahren nach Anspruch 1 oder 2, dadurch gekennzeichnet, dass L¹ 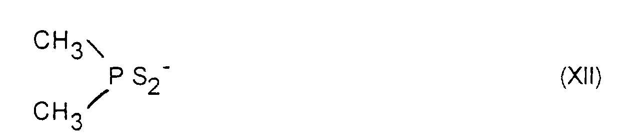 darstellt und L² 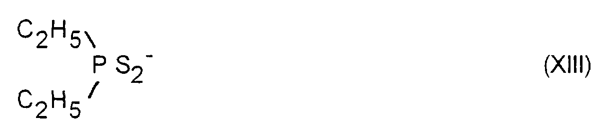 darstellt.

7. Verfahren nach Anspruch 1 oder 2, dadurch gekennzeichnet, dass L¹ und/oder L² der Formel: 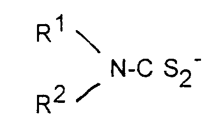 entsprechen, worin R¹ ein durch -COOR³ substituierter Alkylrest und R² ein nicht substituierter oder durch -COOR³ substituierter Alkylrest ist.

8. Verfahren nach Anspruch 1 oder 2, dadurch gekennzeichnet, dass L¹ und/oder L² einer der folgenden Formeln entsprechen: 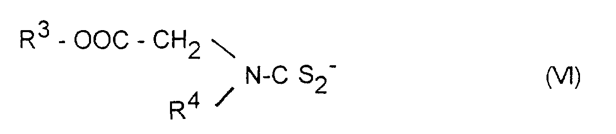 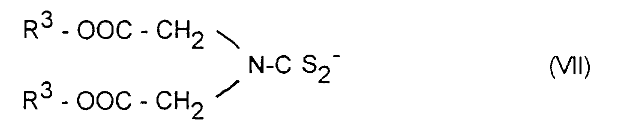 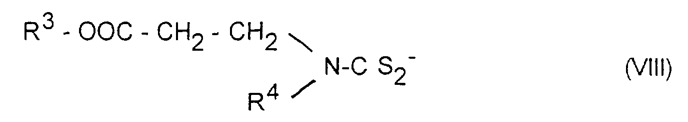 in denen R³ der Methyl- oder Ethylrest ist und R⁴ der Methyl-, Ethyl- oder Propylrest ist.

9. Verfahren nach Anspruch 1 oder 2, dadurch gekennzeichnet, dass L¹ und/oder L² der Formel: 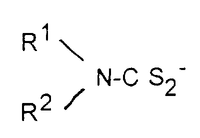 entsprechen, worin R¹ und R² zusammen einen Kohlenwasserstoffring bilden, der durch eine -COOR³-Gruppe substituiert ist, wobei R³ einen linearen oder verzweigten Alkylrest von 1 bis 5 Kohlenstoffatomen darstellt.

10. Verfahren nach einem der Ansprüche 1 bis 9, dadurch gekennzeichnet, dass L¹ und L² gleich sind.

11. Verfahren nach einem der Ansprüche 1 bis 10, dadurch gekennzeichnet, dass M ein Isotop des Technetiums oder des Rheniums darstellt.

12. Verfahren nach Anspruch 11, dadurch gekennzeichnet, dass das Technetiumisotop Tc-99m ist.

13. Verfahren nach Anspruch 11, dadurch gekennzeichnet, dass das Rheniumisotop Re-186 oder Re-188 ist.

14. Verfahren nach Anspruch nach Anspruch 1 oder 2, in welchem das radiopharmazeutische Produkt unter unter Nitrido-bis(dimethyldithiophosphinato)^{99m}Tc(V), Nitrido-bis(diethyldithiophosphinato)^{99m}Tc(V), Nitrido-dimethyldithiophosphinato-diethyldithiophosphinato)^{99m}Tc(V), Nitrido-bis(N-(N,N-dimethylethylamino)-N-methyl-dithiocarbamato)^{99m}Tc(V), Nitrido-bis(N-(N,N-diethylethylamino)-N-methyl-dithiocarbamato)^{99m}Tc(V), Nitrido-bis(N-methoxycarbonylmethyl-N-methyl-dithiocarbamato)^{99m}Tc(V), Nitrido-bis(N-ethoxycarbonylmethyl-N-methyl-dithiocarbamato)^{99m}Tc(V), Nitrido-bis(N-methoxycarbonylmethyl-N-ethyl-dithiocarbamato)^{99m}Tc(V), Nitrido-bis(N-methoxycarbonylmethyl-N-propyl-dithiocarbamato)^{99m}Tc(V), Nitrido-bis(N-ethoxycarbonylmethyl-N-n-propyl-dithiocarbamato)^{99m}Tc(V), Nitrido-bis[(4-methyoxycarbonyl-piperidin-1-yl)-dithiocarbonato]^{99m}Tc(V), Nitrido-bis(bis[N-methoxycarbonylmethyl]-dithiocarbamato)^{99m}Tc(V) und Nitrido-bis[N-(2-methoxycarbonyl-ethyl)-N-methyl-dithiocarbamato]^{99m}Tc(V) gewählt ist.

15. Verfahren nach einem der Ansprüche 1 bis 14, dadurch gekennzeichnet, dass die sauerstoffhaltige Verbindung des Übergangsmetalls ein Pertechnat eines Alkalimetalls oder des Ammoniums ist.

16. Verfahren nach einem der Ansprüche 1 bis 14, dadurch gekennzeichnet, dass die sauerstoffhaltige Verbindung des Übergangsmetalls ein Perrhenat eines Alkalimetalls oder des Ammoniums ist.

17. Verfahren nach Anspruch 1, dadurch gekennzeichnet, dass das erste Reagenz ein Phosphin ist, gewählt unter Triphenylphosphin, Diethylphenylphosphin, Triethylphosphin, Trimethylphosphin, Tris(2-cyanoethyl)phosphin und dem trisulfonierten Triphenylphosphin.

18. Verfahren nach einem der Ansprüche 1 und 2, dadurch gekennzeichnet, dass der stickstoffhaltige Ligand gewählt ist unter S-Methyl-dithiocarbazat, S-Methyl-(2-methyldithiocarbazat), S-Methyl-(1-methyl-2-pyridylmethylendithiocarbazat), S-Methyl-[3-(2'-hydroxyphenyl)methylen-dithiocarbazat] und S-Methyl-[2-methyl-3-(2'-hydroxyphenyl)methylen-dithiocarbazat]..

19. Verfahren nach einem der Ansprüche 1 und 2, dadurch gekennzeichnet, dass der stickstoffhaltige Ligand der Formel: 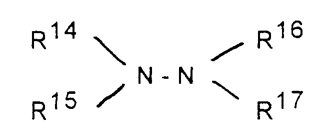 entspricht,
worin R¹⁴, R¹⁵ und R¹⁶, die gleich oder verschieden sein können, ein Wasserstoffatom, einen Alkylrest, einen Arylrest, einen Alkoxyrest, einen Alkylrest, welcher durch mindestens eine unter den Hydroxy-, Carboxy-, Amino-, Amido- und Mercaptoresten gewählte Gruppe substituiert ist, oder einen Arylrest, welcher durch mindestens eine unter den Halogenatomen und den Alkoxy-, Hydroxy-, Amino- und Mercaptoresten und den durch mindestens einen Alkylrest substituierten Aminoresten gewählte. Gruppe substituiert ist, darstellen,
R¹⁷ einen Rest darstellt, der den Formeln:
-SO₂R¹⁸ oder -COR¹⁹
entspricht, worin R¹⁸ eine nicht substituierte oder durch mindestens einen unter den Halogenatomen und den Alkylresten gewählten Substituenten substituierte Phenylgruppe ist und
R¹⁹ ein Wasserstoffatom, -NH₂ oder ein Rest ist, der unter den Alkylresten, den durch mindestens eine unter den Cyano-, Pyridyl- und -CO-NH-NH₂-Gruppen gewählte Gruppe substituierten Alkylresten, dem Phenylrest, dem durch mindestens einen unter -OH, NH₂ und den Alkyl- und Alkoxyresten gewählten Substituenten substituierten Phenylrest gewählt ist.

20. Verfahren nach Anspruch 19, dadurch gekennzeichnet, dass R¹⁴, R¹⁵ und R¹⁶ Wasserstoffatome sind, R¹⁷ den Rest der Formel SO₂R¹⁸, wobei R¹⁸ die in Anspruch 18 gegebene Bedeutung hat, oder den Rest der Formel COH, CO-CH₂-CH₂-CO-NH-NH₂oder 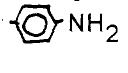 darstellt und dass man die Reaktion bei Umgebungstemperatur ausführt.

21. Verfahren nach Anspruch 20, dadurch gekennzeichnet, dass R¹⁷ den Rest der Formel SO₂R¹⁸ darstellt, wobei R¹⁸ der Phenylrest, der p-Methylphenylrest, der 1,3,5-Trichlorphenylrest oder der 1,3,5-Trimethylphenylrest ist.

22. Verfahren nach einem der Ansprüche 1 und 15 bis 17, dadurch gekennzeichnet, dass das zweite Reagenz Natriumazid ist.

23. Garnitur für die Herstellung eines radiopharmazeutischen Produkts, dadurch gekennzeichnet, dass sie umfasst:
- eine erste Flasche, enthaltend ein Phosphin oder ein Reduktionsmittel, gewählt unter Ammoniumdithionit, den Dithioniten pharmazeutisch annehmbarer Metalle und Zinn(II) in lonenform,
- eine zweite Flasche, enthaltend Ammoniumazid, Azid eines pharmazeutisch annehmbaren Metalls oder einen stickstoffhaltigen Liganden, und
- eine dritte Flasche, enthaltend eine Verbindung entsprechend der Formel: 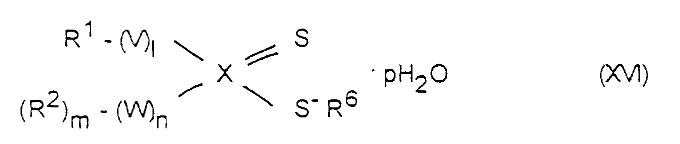 worin R¹, R², V, W, l, m, n und X die in Anspruch 1 gegebene Bedeutung haben, R⁶ ein Alkalimetallion, H⁺ oder NH₄⁺ ist und p gleich 0 oder eine von 1 bis 5 gehende ganze Zahl ist.

24. Verwendung eines durch das Verfahren nach einem der Ansprüche 14 bis 22 erhaltenen Übergangsmetallkomplexes zur Herstellung eines radiopharmazeutischen Produkts mit zerebraler Affinität.

25. Verfahren nach einem der Ansprüche 1 bis 22, dadurch gekennzeichnet, dass das radiopharmazeutische Produkt eine zerebrale Affinität aufweist.

## Claims (Claims for the following Contracting State(s): AT, BE, CH, LI, DE, DK, FR, GB, IT, LU, NL, SE)

1. Radiopharmaceutical product, characterized in that it includes a complex of a transition metal M satisfying the formula:
(M = N) L¹ L² (I)
in which L¹ and L², which may be identical or different satisfy the formula: 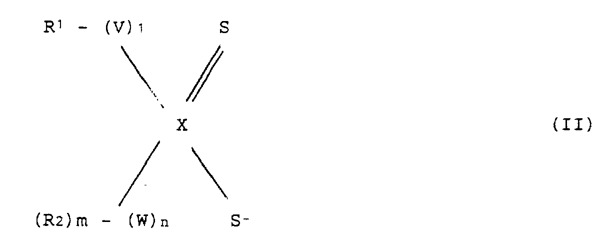 in which R¹ and R², which may be identical or different, represent a linear or branched alkyl radical with 1 to 10 carbon atoms, substituted or not substituted by at least one group selected from the groups -O-R³, -OOCR³, -OCNR⁴R⁵ or -NR⁴R⁵ in which R³ is a linear or branched alkyl radical with 1 to 5 carbon atoms, and R4 and R⁵, which may be identical or different, are hydrogen atoms or linear or branched alkyl radicals with 1 to 5 carbon atoms, or in which R¹ and R² together form a hydrocarbon cycle possibly containing one or several heteroatoms, substituted or not substituted by at least one group selected from the groups -O-R³, -OOCR³, -COOR³, -OCNR⁴R⁵ or -NR⁴R⁵ in which R³, R⁴ and R⁵ are as defined above,
- V and W, which may be identical or different, represent O, S or Se,
- X represents C, N-C, P or As, and
- l, m and n, which may be identical or different, are equal to 0 or 1, provided m and n are equal when X represents C, and m is equal to 1 when X represents N-C, P or As and that R¹ or R² is an alkyl radical substituted by -NR⁴R⁵, -OOCR³ or -COOR³ when X represents N-C, P or As,
provided that
1) when X represents -N-C-, R¹ or R² is an alkyl radical substituted by at least one -COOR³ group, or R¹ and R² together form a substituted hydrocarbon cycle optionally containing one or more heteroatoms and
2) when X represents P with V and W representing 0, R¹ or R² is an alkyl radical substituted by at least one group chosen from among OR³, OOCR³, COOR³, OCNR⁴R⁵ groups with R³, R⁴ and R⁵ as defined hereinbefore, or R¹ and R² together form a substituted hydrocarbon cycle optionally containing one or more heteroatoms.

2. Radiopharmaceutical product according to claim 1, characterized in that L¹ and/or L² satisfy the formula:

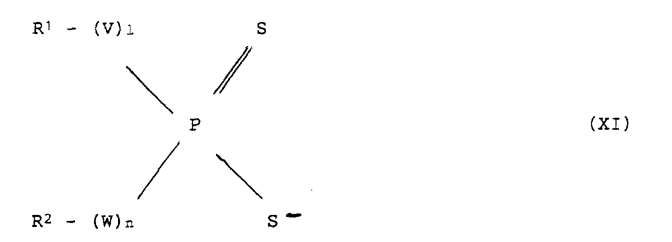

3. Radiopharmaceutical product according to claim 2, characterized in that 1 and n are equal to 0.

4. Radiopharmaceutical product according to claim 3, characterized in that R¹ and R² are methyl or ethyl radicals.

5. Radiopharmaceutical product according to claim 1, characterized in that
L¹ represents 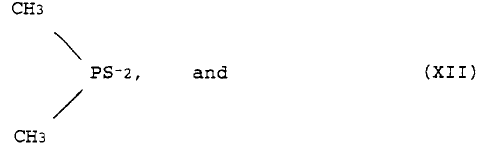
L² represents

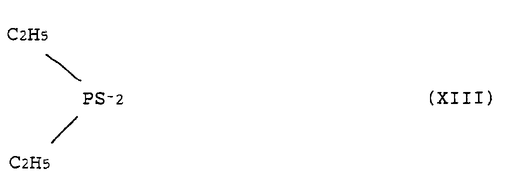

6. Radiopharmaceutical product according to claim 1, characterized in that L¹ and/or L² satisfy the formula: 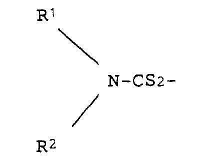 in which R¹ is an alkyl radical substituted by -COOR³ and R² is an alkyl radical substituted or not substituted by -COOR³.

7. Radiopharmaceutical product according to claim 1, characterized in that L¹ and/or L² satisfies one of the following formulae: 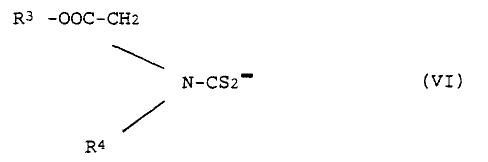 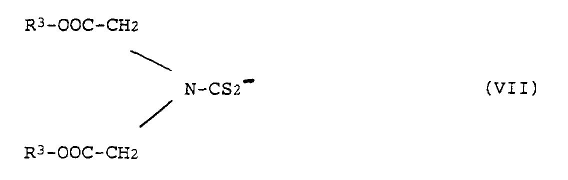 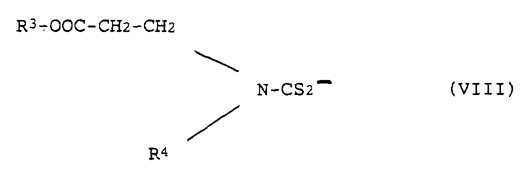 in which R³ is the methyl or ethyl radical and R⁴ is the methyl, ethyl or propyl radical.

8. Radiopharmaceutical product according to claim 1, characterized in that L¹ and/or L² satisfies the formula: 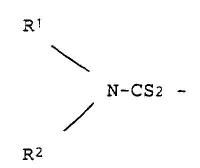 in which R¹ and R² together form a hydrocarbon cycle substituted by a -COOR³ group with R³ representing a linear or branched alkyl radical with 1 to 5 carbon atoms.

9. Radiopharmaceutical product according to any one of the claims 1 to 9, characterized in that L¹ and L² are identical.

10. Radiopharmaceutical product according to any one of the claims 1 to 9, characterized in that M represents an isotope of technetium or rhenium.

11. Radiopharmaceutical product according to claim 10, characterized in that the isotope of technetium is Tc99m.

12. Radiopharmaceutical product according to claim 10, characterized in that the isotope of rhenium is Re-186 or Re-188.

13. Radiopharmaceutical product, characterized in that it is selected from nitruro-bis(dimethyl)dithiophosphinato)^{99m}Tc (V), nitruro-bis (diethyl dithiophosphinato) ^{99m}Tc(V), nitruro(dimethyldithiophosphinato, diethyldithiophosphinato) Tc^{99m}(V), nitruro-bis (N-(N,N dimethylethylamino), N-methyl dithiocarbamato) ^{99m}Tc(V), nitruro-bis (N-(N,N diethylethylamino), N-methyl dithiocarbamato) ^{99m}Tc(V), nitruro-bis (N-methylenemethylcarboxylate, N-methyl dithiocarbamato) ^{99m}Tc(V), nitruro-bis (N-methylene ethyl carboxylate, N-methyl dithiocarbamato) ^{99m}Tc(V), nitruro-bis (N-methylene methyl carboxylate, N-ethyl dithiocarbamato) ^{99m}Tc(V), nitruro-bis (N-methylene methyl carboxylate, N-propyl dithiocarbamato) ^{99m}Tc(V), nitruro-bis (N-methylene ethyl carboxylate, N-n-propyl dithiocarbamato) ^{99m}Tc(V), nitruro-bis (3-(methyl carboxylate)piperidino dithiocarbamato) ^{99m}Tc(V), nitruro-bis [bis N-(methylene methyl carboxylate] dithiocarbamato) ^{99m}Tc(V), and nitruro-bis (N-ethylene-2-(methyl carboxylate), N-methyl dithiocarbamato) ^{99m}Tc(V).

14. Method for preparing a radiopharmaceutical product according to any one of claims 1 to 13, characterized in that it includes the following successive stages:
1) making an oxygenous compound of a transition metal M in a solution react with:
a) a first reactive agent selected from the group of aliphatic and aromatic phosphines and polyphosphines, possibly substituted, and
b) a second reactive agent selected from pharmaceutically acceptable ammonium and metal nitrides and nitrogenous ligands comprising a 〉N-N〈 pattern in which the Ns are linked to hydrogen atoms and/or monovalent organic groups, or in which one of the Ns is linked to the carbon atom of a bivalent organic group by means of a double link, the other N being linked to hydrogen atoms and/or monovalent organic groups, and
2) making the intermediate product obtained in the first stage react with a compound satisfying the formula: 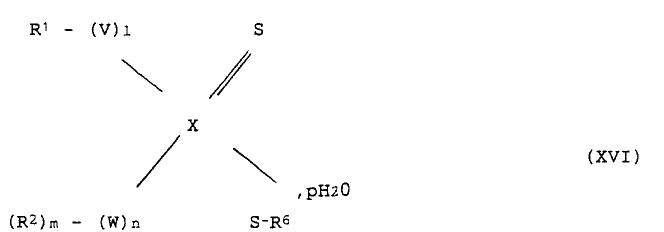 in which R¹, R², V, W, X, l, m and n have the meanings given in claim 1, R⁶ being an alkali metal ion, H⁺ or NH₄⁺, and p being equal to 0 or a whole number from 1 to 5.

15. Method for preparing a radiopharmaceutical product according to any one of claims 1 to 13, characterized in that it includes the following stages:
1) making an oxygenous compound of a transition metal M in a solution react with
a) a nitrogenous ligand constituted by either a pharmaceutically acceptable metal or ammonium nitride, or by a nitrogenous compound comprising a 〉N-N〈 pattern in which the Ns are linked to hydrogen atoms and/or monovalent organic groups, or in which one of the Ns is linked to the carbon atom of a bivalent organic group by means of a double link, the other N〈 being linked to hydrogen atoms and/or monovalent organic groups, and
b) a reducing agent constituted by either ammonium dithionite or a pharmaceutically acceptable metal, or by tin (II) present in an ionic form in the solution, and
2) making the intermediate product obtained in the first stage react with a compound satisfying the formula: 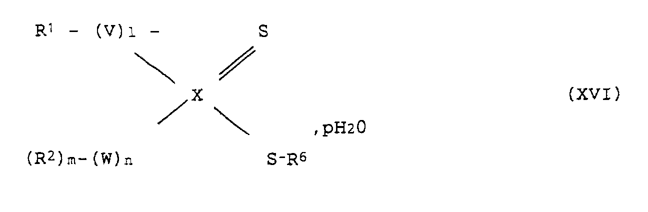 in which R¹, R², V,W, l, m, n and X have the meanings given in claim 1, R⁶ being an alkali metal ion, H⁺ or NH₄⁺, and p being equal to 0 or a whole number from 1 to 5.

16. Method according to claim 14 or 15, characterized in that the oxygenous compound of the transition metal is an ammonium or alkali metal pertechnetate.

17. Method according to claim 14 or 15, characterized in that the oxygenous compound of the transition metal is ammonium or alkali metal perrhenate.

18. Method according to claim 14, characterized in that the first reactive agent is a phosphine selected from triphenylphosphine, diethylphenylphosphine triethylphosphine, trimethylphosphine, tris(2-cyanoethyl)phosphine and trisulphonated triphenylphosphine.

19. Method according to claim 14 or 15, characterized in that the nitrogenous ligand is selected from S-methyldithiocarbazate, S-methyl-N-methyldithiocarbazate, alpha-N-methyl-S-methyl beta-N-pyridylmethylene dithiocarbazate, S-methyl-beta-N-(2-hydroxyphenyl)methylene dithiocarbazate and alpha-N-methyl-S-methyl-beta-N-(2-hydroxyphenyl)methylene dithiocarbazate.

20. Method according to claim 14 or 15, characterized in that the nitrogenous ligand satisfies the formula: 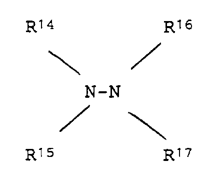
in which R¹⁴, R¹⁵ and R¹⁶, which may be identical or different represent an atom of hydrogen, an alkyl radical, an aryl radical, an alkoxy radical, an alkyl radical substituted by at least one group selected from the hydroxy carboxy, amino, amido and mercapto radicals or an aryl radical substituted by at least one group selected from the halogen atoms and the alkoxy, hydroxy, amino, mercapto and amino radicals substituted by at least one alkyl radical, R¹⁷ being a radical satisfying the formula:
-SO₂R¹⁸ or -COR¹⁹
in which R¹⁸ is a phenyl group, substituted or not substituted by at least one substituent selected from halogen atoms and alkyl radicals, and R¹⁹ is a hydrogen atom, -NH₂ or a radical selected from the alkyl radicals, the alkyl radicals substituted by at least one group selected from the cyano, pyridyl and -CO-NH-NH₂ groups, the phenyl radical, the phenyl radical substituted by at least one substituent selected from -OH, NH₂ and the alkoxy and alkyl radicals.

21. Method according to claim 20, characterized in that R¹⁴, R¹⁵ and R¹⁶ are hydrogen atoms, R¹⁷ represents the radical of formula SO₂R¹⁸ with R¹⁸ having the meaning given in claim 18, or the radical of formula COH, CO-CH₂-CH₂-CO-NH-NH₂ or 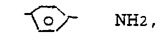 and in that the reaction is carried out at ambient temperature.

22. Method according to claim 21, characterized in that R¹⁷ represents SO₂R¹⁸ with R¹⁸ being the phenyl radical, the p-methylphenyl radical, the 1,3,5-trichlorophenyl radical or the 1,3,5 trimethylphenyl radical.

23. Method according to any one of the claims 14 and 16 to 18, characterized in that the second reactive agent is sodium nitride.

24. Kit for preparing a radiopharmaceutical product, characterized in that it includes:
- a first flask containing a phosphine or reducing agent selected from ammonium dithionite, pharmaceutically acceptable metal dithionites and tin (II) in an ionic form,
- a second flask containing ammonium nitride, the nitride of a pharmaceutically acceptable metal, or a nitrogenous ligand, and
- a third flask containing a compound satisfying the formula: 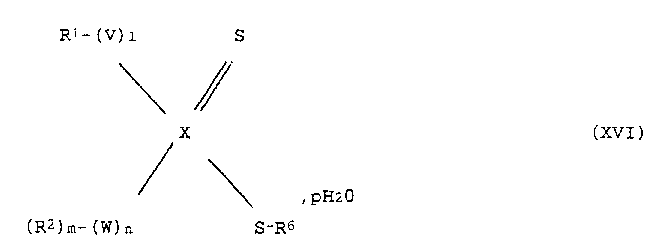 in which R¹, R², V, W, l, m, n and X have the meanings given in claim 1, R⁶ being an alkali metal ion, H⁺ or NH4⁺, and p being equal to 0 or a whole number of between 1 and 5.

25. Use of a transition metal complex obtained by the method according to any one of claims 14 to 23 for producing a radiopharmaceutical product with cerebral tropism.

26. Pharmaceutical product according to any one of the claims 1 to 13, characterized in that it has a cerebral tropism.

## Claims (Claims for the following Contracting State(s): ES, GR)

1. Radiopharmaceutical product, characterized in that it includes a complex of a transition metal M satisfying the formula:
(M = N) L¹ L² (I)
in which L¹ and L², which may be identical or different satisfy the formula: 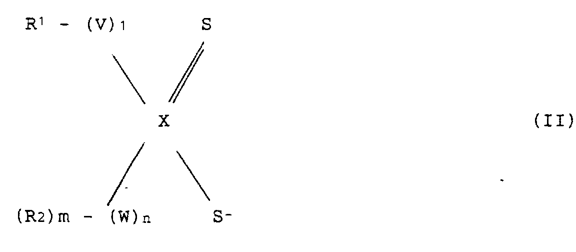 in which R¹ and R², which may be identical or different, represent a linear or branched alkyl radical with 1 to 10 carbon atoms, substituted or not substituted by at least one group selected from the groups -O-R³, -OOCR³, -OCNR⁴R⁵ or -NR⁴R⁵ in which R³ is a linear or branched alkyl radical with 1 to 5 carbon atoms, and R⁴ and R⁵, which may be identical or different, are hydrogen atoms or linear or branched alkyl radicals with 1 to 5 carbon atoms, or in which R¹ and R² together form a hydrocarbon cycle possibly containing one or several heteroatoms, substituted or not substituted by at least one group selected from the groups -O-R³, -OOCR³, -COOR³, -OCNR⁴R⁵ or -NR⁴R⁵ in which R³, R⁴ and R⁵ are as defined above,
- V and W, which may be identical or different, represent O, S or Se,
- X represents C, N-C, P or As, and
- l, m and n, which may be identical or different, are equal to 0 or 1, provided m and n are equal when X represents C, and m is equal to 1 when X represents N-C, P or As and that R¹ or R² is an alkyl radical substituted by -NR⁴R⁵ -OOCR³ or -COOR³ when X represents N-C, P or As,
provided that
1) when X represents -N-C-, R¹ or R² is an alkyl radical substituted by at least one -COOR³ group, or R¹ and R² together form a substituted hydrocarbon cycle optionally containing one or more heteroatoms and
2) when X represents P with V and W representing 0, R¹ or R² is an alkyl radical substituted by at least one group chosen from among OR³, OOCR³, COOR³, OCNR⁴R⁵ groups with R³, R⁴ and R⁵ as defined hereinbefore, or R¹ and R² together form a substituted hydrocarbon cycle optionally containing one or more heteroatoms.
1) reacting in solution an oxygen compound of the transmission metal M with:
a) a first reagent chosen from within the group of aliphatic and aromatic phosphines and polyphosphines, which may or may not be substituted, and
b) a second reagent chosen from among pharmaceutically acceptable metals and ammonium nitrides and nitrogenous ligands having a 〉N-N〈 pattern, in which the N are connected to hydrogen atoms and/or monovalent organic groups, or in which one of the N is connected to the carbon atom of a divalent organic group by means of a double bond and the other N is connected to hydrogen atoms and/or to monovalent organic groups and
2) reacting the intermediate product obtained in the first stage with a compound satisfying the formula: 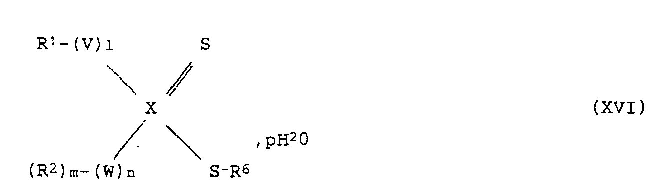 in which R¹, R², V, W, X, l, m and n have the meanings given herein above and R⁶ is an alkali metal ion, H⁺ or NH₄⁺ and p is equal to 0 or a whole number from 1 to 5.

2. Process for the preparation of a radiopharmaceutical product including a complex of a transition metal M satisfying the formula:
(M ≡ N) L¹ L² (I)
in which L¹ and L², which may be identical or different satisfy the formula: 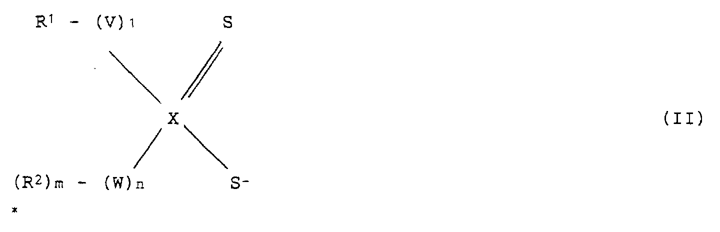 in which R¹ and R², which may be identical or different, represent a linear or branched alkyl radical with 1 to 10 carbon atoms, substituted or not substituted by at least one group selected from the groups -O-R³, -OOCR³, -OCNR⁴R⁵ or -NR⁴R⁵ in which R³ is a linear or branched alkyl radical with 1 to 5 carbon atoms, and R⁴ and R⁵, which may be identical or different, are hydrogen atoms or linear or branched alkyl radicals with 1 to 5 carbon atoms, or in which R¹ and R² together form a hydrocarbon cycle possibly containing one or several heteroatoms, substituted or not substituted by at least one group selected from the groups -O-R³ -OOCR³, COOR³, -OCNR⁴R⁵ or -NR⁴R⁵ in which R³, R⁴ and R⁵ are as defined above,
- V and W, which may be identical or different, represent O, S or Se,
- X represents C, N-C, P or As, and
- l, m and n, which may be identical or different, are equal to 0 or 1, provided m and n are equal to 0 when X represents C, and m is equal to 1 when X represents N-C, P or As and the R¹ or R² is an alkyl radical substituted by -NR⁴R⁵ -OOCR³ when X represents N-C, P or As,
provided that
1) when X represents -N-C, R¹ or R² is an alkyl radical substituted by at least one -COOR3 group, or R¹ and R² together form a substituted hydrocarbon cycle optionally containing one or more heteroatoms and
2) when X represents P with V and W representing 0, R¹ or R² is an alkyl radical substituted by at least one group chosen from among OR³, OOCR³ COOR³, OCNR⁴R⁵ groups with R³, R⁴ and R⁵ as defined hereinbefore, or R¹ and R² together form a substituted hydrocarbon cycle optionally containing one or more heteroatoms optionally containing one or more heteroatoms, said process including the following stages:
1) reacting in solution an oxygen compound of the transition metal M with
a) a nitrogen ligand constituted either by a pharmaceutically acceptable metal or an ammonium nitride, either by a nitrogen compound having a 〉N-N〈 pattern, in which the N are connected to hydrogen atoms and/or to monovalent organic groups, or in which one of the N is connected to the carbon atom of a divalent organic group by means of a double bond and the other N〈 is connected to hydrogen atoms and/or monovalent organic groups, and
b) a reducing agent constituted either by a pharmaceutically acceptable metal or ammonium dithionite, or by tin (II) present in ionic form in the solution, and
2) reacting the intermediate product obtained in the first stage with a compound satisfying the formula 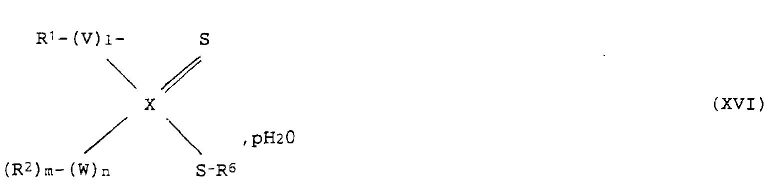 in which R¹, R², V, W, l, m, n and X have the meanings given herein above, R⁶ is an alkali metal ion, H⁺ or NH₄⁺ and p is equal to 0 or a whole number from 1 to 5.

3. Process according to claim 1 or 2, characterized in that L¹ and/or L² satisfy the formula:

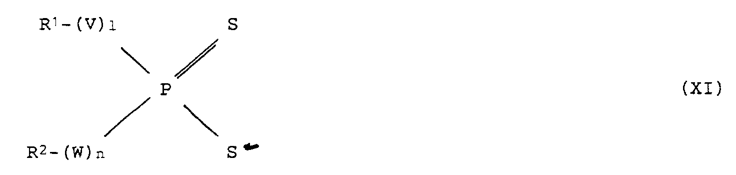

4. Process according to claim 3, characterized in that 1 and n are equal to 0.

5. Process according to claim 4, characterized in that R¹ and R² are methyl or ethyl radicals.

6. Process according to claim 1 or 2, characterized in that
L¹ represents 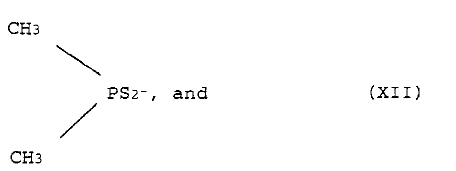
L² represents

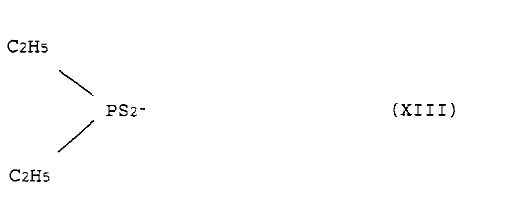

7. Process according to claim 1 or 2, characterized in that L¹ and/or L² satisfy the formula: 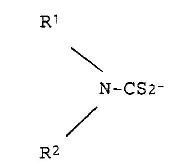 in which R¹ is an alkyl radical substituted by -COOR³ and R² is an alkyl radical which may or may not be substituted by -COOR³.

8. Process according to claim 1 or 2, characterized in that L¹ and/or L² satisfy one of the following formulas: 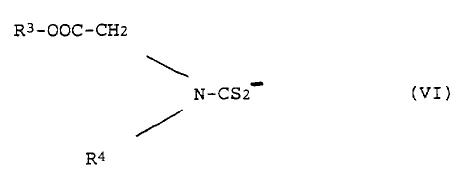 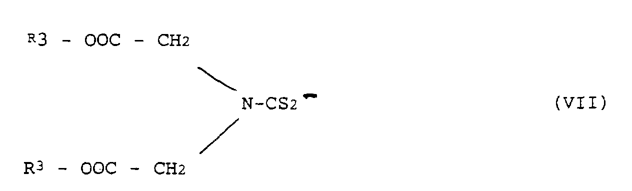 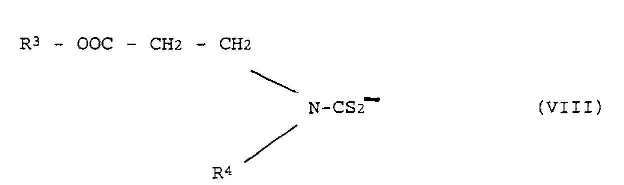 in which R³ is a methyl or ethyl radical and R⁴ is the methyl, ethyl or propyl radical.

9. Process according to claim 1 or 2, characterized in that L¹ and/or L² satisfy the formula: 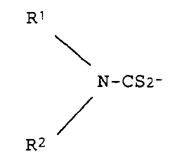 in which R¹ and R² together form a hydrocarbon cycle substituted by a -COOR³ group with R³ representing a straight or branched alkyl radical with 1 to 5 carbon atoms.

10. Process according to any one of the claims 1 to 9, characterized in that L¹ and L² are identical.

11. Process according to any one of the claims 1 to 10, characterized in that M represents an isotope of technetium or rhenium.

12. Process according to claim 11, characterized in that the isotope of technetium is Tc99m.

13. Process according to claim 11, characterized in that the isotope of rhenium is Re-186 or Re-188.

14. Process according to claim 1 or 2, in which the radiopharmaceutical product is selected from nitruro-bis(dimethyl)dithiophosphinato)^{99m}Tc(V), nitruro-bis (diethyl dithiophosphinato) ^{99m}Tc(V), nitruro(dimethyldithiophosphinato), diethyldithiophosphinato) Tc^{99m}(V), nitruro-bis (N-(N,N dimethylethylamino), N-methyl dithiocarbamato) ^{99m}Tc(V), nitruro-bis (N-(N,N diethylethylamino), N-methyl dithiocarbamato) ^{99m}Tc(V), nitruro-bis (N-methylenemethylcarboxylate, N-methyl dithiocarbamato) ^{99m}Tc(V), nitruro-bis (N-methylene ethyl carboxylate, N-methyl dithiocarbamato) ^{99m}Tc(V), nitruro-bis (N-methylene methyl carboxylate, N-ethyl dithiocarbamato) ^{99m}Tc(V), nitruro-bis (N-methylene methyl carboxylate, N-propyl dithiocarbamato) ^{99m}Tc(V), nitruro-bis (N-methylene ethyl carboxylate, N-n-propyl dithiocarbamato) ^{99m}Tc(V), nitruro-bis (3-(methyl carboxylate)piperidino dithiocarbamato) ^{99m}Tc(V), nitruro-bis [bis N-methylene methyl carboxylate] dithiocarbamato) ^{99m}Tc(V), and nitruro-bis (N-ethylene-2-(methyl carboxylate), N-methyl dithiocarbamato) ^{99m}Tc(V).

15. Process according to any one of the claims 1 to 14, characterized in that the oxygen compound of the transition metal is an ammonium or alkali metal pertechnetate.

16. Process according to any one of the claims 1 to 14, characterized in that the oxygen compound of the transition metal is ammonium or alkali metal perrhenate.

17. Process according to claim 1, characterized in that the first reactive agent is a phosphine selected from triphenylphosphine, diethylphenylphosphine triethylphosphine, trimethylphosphine, tris(2-cyanoethyl)phosphine and trisulphonated triphenylphosphine.

18. Process according to either of the claims 1 and 2, characterized in that the nitrogenous ligand is selected from S-methyldithiocarbazate, S-methyl-N-methyldithiocarbazate, alpha-N-methyl-S-methyl beta-N-pyridylmethylene dithiocarbazate, S-methyl-beta-N-(2-hydroxyphenyl)methylene dithiocarbazate and alpha-N-methyl-S-methyl-beta-N-(2-hydroxyphenyl)methylene dithiocarbazate.

19. Process according to either of the claims 1 and 2, characterized in that the nitrogenous ligand satisfies the formula: 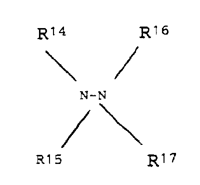 in which R¹⁴, R¹⁵ and R¹⁶, which may be identical or different represent an atom of hydrogen, an alkyl radical, an aryl radical, an alkoxy radical, an alkyl radical substituted by at least one group selected from the hydroxy carboxy, amino, amido and mercapto radicals or an aryl radical substituted by at least one group selected from the halogen atoms and the alkoxy, hydroxy, amino, mercapto and amino radicals substituted by at least one alkyl radical, R¹⁷ being a radical satisfying the formula:
-SO₂R¹⁸ or COR¹⁹
in which R¹⁸ is a phenyl group, substituted or not substituted by at least one substituent selected from halogen atoms and alkyl radicals, and R¹⁹ is a hydrogen atom, -NH₂ or a radical selected from the alkyl radicals, the alkyl radicals substituted by at least one group selected from the cyano, pyridyl and -CO-NH-NH₂ groups, the phenyl radical, the phenyl radical substituted by at least one substituent selected from -OH, NH₂ and the alkoxy and alkyl radicals.

20. Process according to claim 19, characterized in that R¹⁴, R¹⁵ and R¹⁶ are hydrogen atoms, R¹⁷ represents the radical of formula SO₂R¹⁸ with R¹⁸ having the meaning given in claim 18, or the radical of formula COH, CO-CH₂-CH₂-CO-NH-NH₂ or 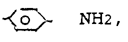 and in that the reaction is carried out at ambient temperature.

21. Process according to claim 20, characterized in that R¹⁷ represents SO₂R¹⁸ with R¹⁸ being the phenyl radical, the p-methylphenyl radical, the 1,3,5-trichlorophenyl radical or the 1,3,5-trimethylphenyl radical.

22. Process according to any one of the claims 1 and 15 to 17, characterized in that the second reagent is sodium nitride.

23. Kit for preparing a radiopharmaceutical product, characterized in that it includes:
- a first flask containing a phosphine or reducing agent selected from ammonium dithionite, pharmaceutically acceptable metal dithionites and tin (II) in an ionic form,
- a second flask containing ammonium nitride, the nitride of a pharmaceutically acceptable metal, or a nitrogenous ligand, and
- a third flask containing a compound satisfying the formula: 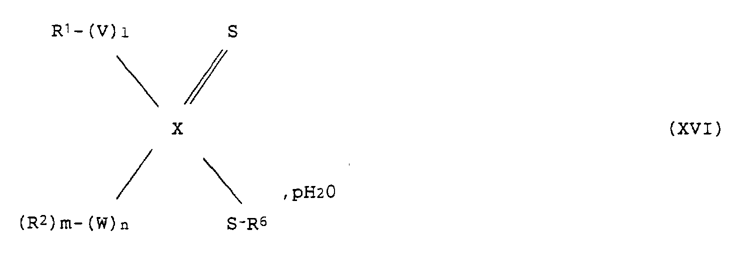 in which R¹, R2, V, W, 1, m, n and X have the meanings given in claim 1, R⁶ being an alkali metal ion, H⁺ or NH4⁺, and p being equal to 0 or a whole number of between 1 and 5.

24. Use of a transition metal complex obtained by the method according to any one of the claims 14 to 22 for producing a radiopharmaceutical product with cerebral tropism.

25. Method according to any one of the claims 1 to 22, wherein the radiopharmaceutical product has a cerebral tropism.
